(19) Europäisches Patentamt
European Patent Office
Office européen des brevets

(11) Veröffentlichungsnummer: **0 019 899**
**B1**

(12) # EUROPÄISCHE PATENTSCHRIFT

(45) Veröffentlichungstag der Patentschrift: **18.07.84**

(51) Int. Cl.[3]: **C 07 D 211/46,** C 07 D 211/56, A 61 K 31/445, A 23 K 1/16

(21) Anmeldenummer: **80102943.0**

(22) Anmeldetag: **27.05.80**

(54) **Derivate des 3,4,5-Trihydroxypiperidins, Verfahren zu ihrer Herstellung und ihre Verwendung als Arzneimittel sowie in der Tierernährung.**

(30) Priorität: **05.06.79 DE 2922760**

(43) Veröffentlichungstag der Anmeldung: **10.12.80 Patentblatt 80/25**

(45) Bekanntmachung des Hinweises auf die Patenterteilung: **18.07.84 Patentblatt 84/29**

(84) Benannte Vertragsstaaten: **AT BE CH DE FR GB IT LI LU NL SE**

(56) Entgegenhaltungen: **EP - A - 0 000 947**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Böshagen, Horst, Dr., Wiesenstrasse 4, D-5657 Haan (DE)**
Erfinder: **Junge, Bodo, Dr., Wilkhausstrasse 123, D-5600 Wuppertal 2 (DE)**
Erfinder: **Stoltefuss, Jürgen, Ing.-grad., Parkstrasse 20, D-5657 Haan (DE)**
Erfinder: **Schmidt, Delf, Dr., Am Eckbusch 55b, D-5600 Wuppertal 1 (DE)**
Erfinder: **Krause, Hans Peter, Dr., Wilkhausstrasse 107, D-5600 Wuppertal 2 (DE)**
Erfinder: **Puls, Walter, Dr., In den Birken 75, D-5600 Wuppertal (DE)**

**EP 0 019 899 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue Derivate des 3,4,5-Trihydroxypiperidins, Verfahren zu ihrer Herstellung sowie ihre Verwendung als Arzneimittel, insbesondere als Mittel gegen Diabetes, Hyperlipämie und Adipositas, sowie in der Tierernährung zur besseren Futterverwertung und zur Beeinflussung des Fleisch/Fettverhältnisses zugunsten des Fleischanteils.

Die neuen Verbindungen entsprechen der Formel I

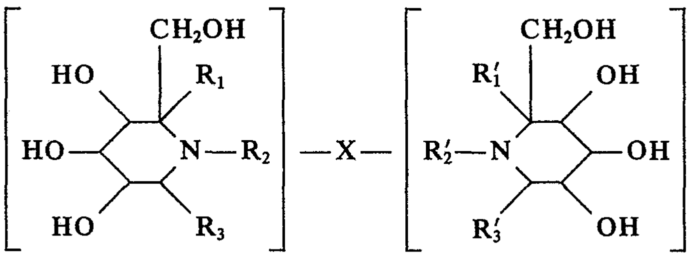

(I)

worin

$R_1$, $R'_1$, $R_3$ und $R'_3$ Wasserstoff oder eine direkte Bindung zu X,

$R_2$ und $R'_2$ Wasserstoff, $C_1-C_4$Alkyl oder eine direkte Bindung zu X bedeuten, mit der Maßgabe, daß einer und nur einer der Reste $R_1$, $R_2$ und $R_3$ und einer und nur einer der Reste $R'_1$, $R'_2$, und $R'_3$ eine direkte Bindung zu X darstellen und

worin X der Formel

$$-(A)_m-(R_4)_n-(Y)_p-(R_5)_q-(B)_r-$$

entspricht, worin

m, n, p, q und r 0 oder 1 bedeuten, mit der Maßgabe, daß die Summe aus m und r 1 oder 2 ist und daß p 0 ist, wenn n und/oder q 0 sind und worin

A und B für

$CH_2$ $CH_2-NH$ $CH_2-NH-CO$ $CH_2-NHCONH$ $CH_2NHSO_2NH$

$CH_2-NH-SO_2$ $CH_2-NHCS-NH$ $CH_2-NH-COO$ $CH_2-NHCS$

$CH_2-NH-C(=NH)-NH$ $CH_2-O$ $CO$ oder $COO$

stehen,

$R_4$ und $R_5$ unabhängig voneinander eine Gruppierung

$$-(R_{13})_s-(U)_t-(R_{14})_v$$

darstellen, in der $R_{13}$ und $R_{14}$ unabhängig voneinander $C_1-C_{18}$ Alkylen; $C_2-C_{18}$ Alkenylen mit bis zu drei Doppelbindungen; $C_2-C_{10}$-Alkinylen; $C_3-C_{10}$-Cycloalkylen; Naphthylen, Phenylen; Phenylen mit einem oder zwei Substituenten aus der Gruppe Phenoxy oder Methyl, einen heterocyclischen Rest mit 3 bis 8 Ringgliedern und 1 bis 4 Heteroatomen N, O und/oder S oder Kombinationen dieser Bedeutung darstellen,

U die Bedeutung von O, S, SO, $SO_2$, NH, CO, COO, CS, OCOO, NHCOO, CONH, NHCONH, NHCSNH, CH=N, $SO_2NH$ oder $NHSO_2NH$ hat,

s, t, v unabhängig voneinander für 0 oder 1 stehen,

Y die Bedeutung von U oder die Bedeutung von $R_{13}$ und $R_{14}$ hat.

Die heterocyclischen Reste $R_{13}$ und $R_{14}$ sind bevorzugt von heteroparaffinischen, heteroaromatischen oder heteroolefinischen 5- oder 6gliedrigen Ringen mit vorzugsweise 1 bis 3 gleichen oder verschiedenen Heteroatomen abgeleitet. Als Heteroatome stehen Sauerstoff, Schwefel oder Stickstoff. Diese Ringsysteme können weitere Substituenten wie beispielsweise Hydroxy-, Amino- oder $C_1-C_4$-Alkylgruppen tragen oder an sie können Benzolkerne oder weitere vorzugsweise 6gliedrige heterocyclische Ringe der genannten Art anneliert sein.

Besonders bevorzugte heterocyclische Reste leiten sich beispielsweise von Furan, Pyran, Pyrrolidon, Piperidin, Pyrazol, Imidazol, Pyrimidin, Pyridazin, Pyrazin, Triazin, Pyrrol, Pyridin, Benzimidazol, Chinolin, Isochinolin oder Purin ab.

Bevorzugte Verbindungen im Sinne der Erfindung sind diejenigen Verbindungen der Formel I, in denen beide 2-Hydroxymethyl-3,4,5-trihydroxypiperidylreste durch die stereochemische Formel II

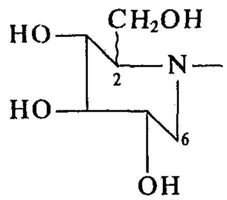

(II)

beschrieben werden und das Brückenglied X wahrscheinlich axial angeordnet ist.

Die Festlegung der Konfiguration an den C-Atomen 2 und 6 stützt sich auf spektroskopische Untersuchungen und ist nicht mit letzter Sicherheit bewiesen.

Es wurde gefunden, daß die neuen Verbindungen der Formel I potente Inhibitoren für $\alpha$-Glucosidasen, insbesondere für Disaccharidasen sind. Daher sind die neuen Verbindungen wertvolle Mittel zur Beeinflussung einer Vielzahl von Stoffwechselvorgängen und bereichern somit den Arzneimittelschatz.

Die erfindungsgemäßen Verbindungen lassen sich nach verschiedenen an sich bekannten Verfahren herstellen.

Ein Verfahren ist dadurch gekennzeichnet, daß man Verbindungen der Formel III

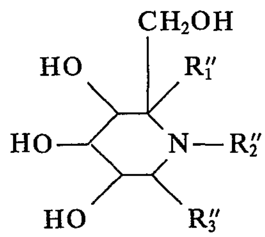

(III)

worin

$R_1''$ und $R_3''$ Wasserstoff oder $CH_2-NH_2$ und

$R_2''$ Wasserstoff oder $C_1-C_4$Alkyl bedeuten, wobei die Zahl der $CH_2-NH_2$ 0 oder 1 ist und $R_2''$ Wasserstoff bedeutet, falls $R_1''$ und $R_3''$ Wasserstoff sind,

mit Dialdehyden der Formel IV

$$OCH—Z—CHO \qquad (IV)$$

worin

Z die zum Brückenglied X fehlenden Glieder bedeutet,

im Verhältnis 2 : 1 in Gegenwart eines Wasserstoffdonors umsetzt.

Setzt man Pentan-1,5-dial und 1-Desoxynojirimycin in die Reaktion ein, so läßt sich der Reaktionsablauf wie folgt wiedergeben:

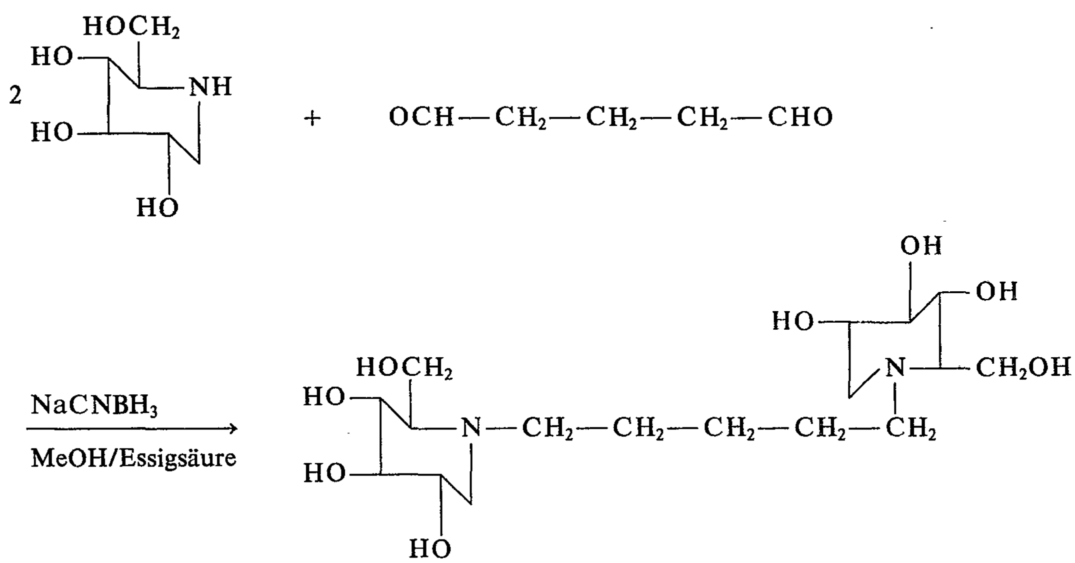


Ein weiteres Verfahren ist dadurch gekennzeichnet, daß man Verbindungen der Formel III mit Alkylierungs- und Acylierungsmitteln der Formel V

$$R_7—Z—R_7 \quad (V)$$

worin

Z die obengenannte Bedeutung hat und
$R_7$ eine in Alkylierungs- und Acylierungsmitteln übliche funktionelle Gruppe, beispielsweise ein Halogenid- oder ein Carbonsäurechloridrest ist, umsetzt.

Verbindungen der Formel V gehören beispielsweise zu folgenden Stoffklassen: Alkylhalogenide, Sulfonsäureester, Carbonsäurehalogenide, Sulfonsäurehalogenide, Chlorkohlensäureester, Isocyanate, Isothiocyanate und Olefine mit aktivierten Doppelbindungen, wobei die Verbindungen der Formel V zwei, gegebenenfalls unterschiedliche, reaktionsfähige Gruppen aufweisen und im Verhältnis 1 : 2 mit den Verbindungen der Formel III umgesetzt werden.

Mit Sebacinsäurechlorid als Ausgangsstoff läßt sich die Reaktion beispielsweise wie folgt formulieren:

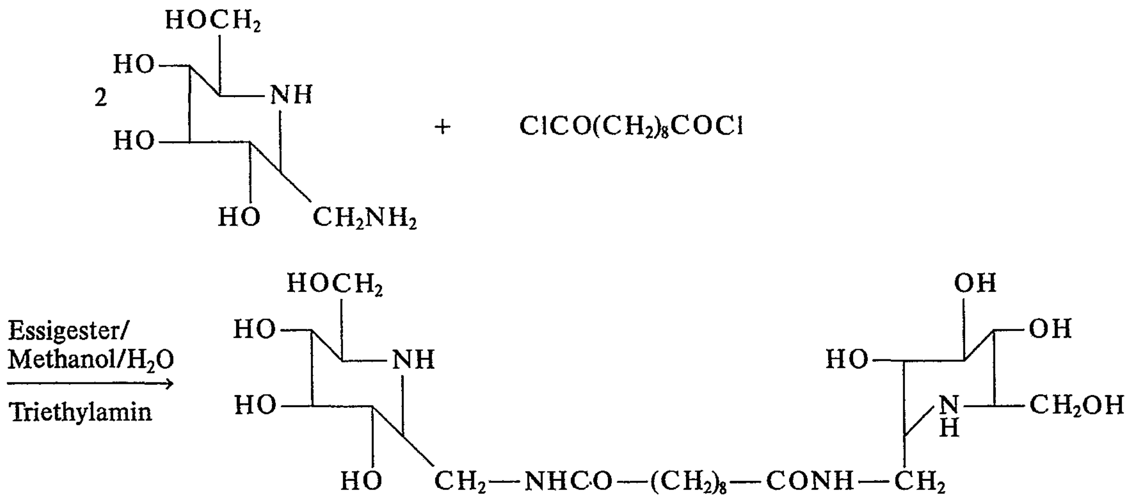


Ein weiteres Verfahren ist dadurch gekennzeichnet, daß man Verbindungen der Formel VI

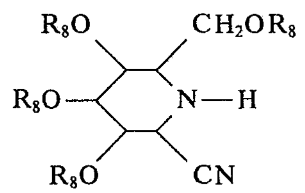
(VI)

worin
$R_8$ eine übliche Schutzgruppe, z. B. ein Trimethylsilylrest ist,
mit Verbindungen der Formel VII

$$BrMg - Z' - MgBr \quad (VII)$$

worin
Z' eine Alkylengruppe oder Phenylengruppe ist,
unter Abspaltung der Cyangruppe und anschließender, vorzugsweise hydrolytischer Entfernung der Schutzgruppen, umsetzt.

Verbindungen der Formel I werden auch erhalten, wenn die Verknüpfung der zwei Trihydroxypiperidineinheiten in mehreren unterschiedlichen Schritten durchgeführt wird, wobei die zweite funktionelle Gruppe des Brückengliedes erst nach der Verknüpfung an eine Verbindung der Formel III aus einer inaktiven Form gebildet wird oder die zweite funktionelle Gruppe mit einer unterschiedlichen Reaktivität erst unter veränderten Reaktionsbedingungen mit einer zweiten Verbindung der Formel III reagiert.

Ein solches Verfahren ist dadurch gekennzeichnet, daß man Verbindungen der Formel III im Verhältnis 1 : 1 mit Verbindungen der Formel VIII

$$R_9—Z—R_{10} \quad (VIII)$$

wobei

$R_9$ — CHO oder $R_7$ und Z die bereits genannte Bedeutung haben und
$R_{10}$ $R_7$ oder eine in einen Rest $R_7$ überführbare Gruppe oder eine Aldehyd- oder Ketogruppe ist,

gegebenenfalls in Gegenwart eines Wasserstoffdonors umsetzt und den Rest $R_{10}$ im Zwischenprodukt unter veränderten Reaktionsbedingungen z. B. unterschiedliche Temperatur, unterschiedliche Lösungsmittel oder in Gegenwart von Säuren und Salzen oder nach Umwandlung in einen Rest $R_7$ mit einer weiteren Verbindung der Formel III gegebenenfalls in Gegenwart eines Wasserstoffdonors zur Reaktion bringt.

Dies sei an folgenden Beispielen erläutert:

Addiert man 1-Desoxynojirimycin an Acrylnitril, so erhält man die N-alkylierte Verbindung, die sich zur Aminoverbindung hydrieren oder zur Carbonsäure verseifen läßt.

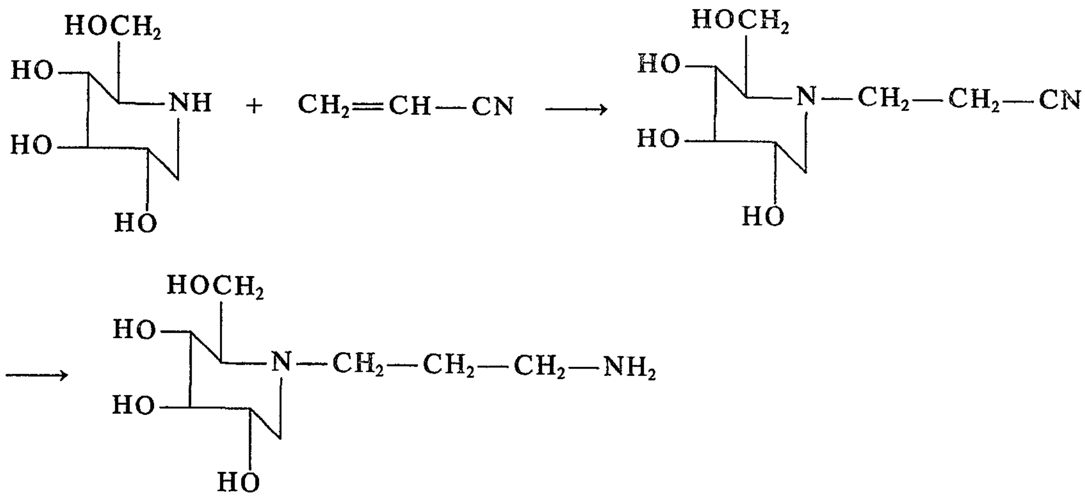

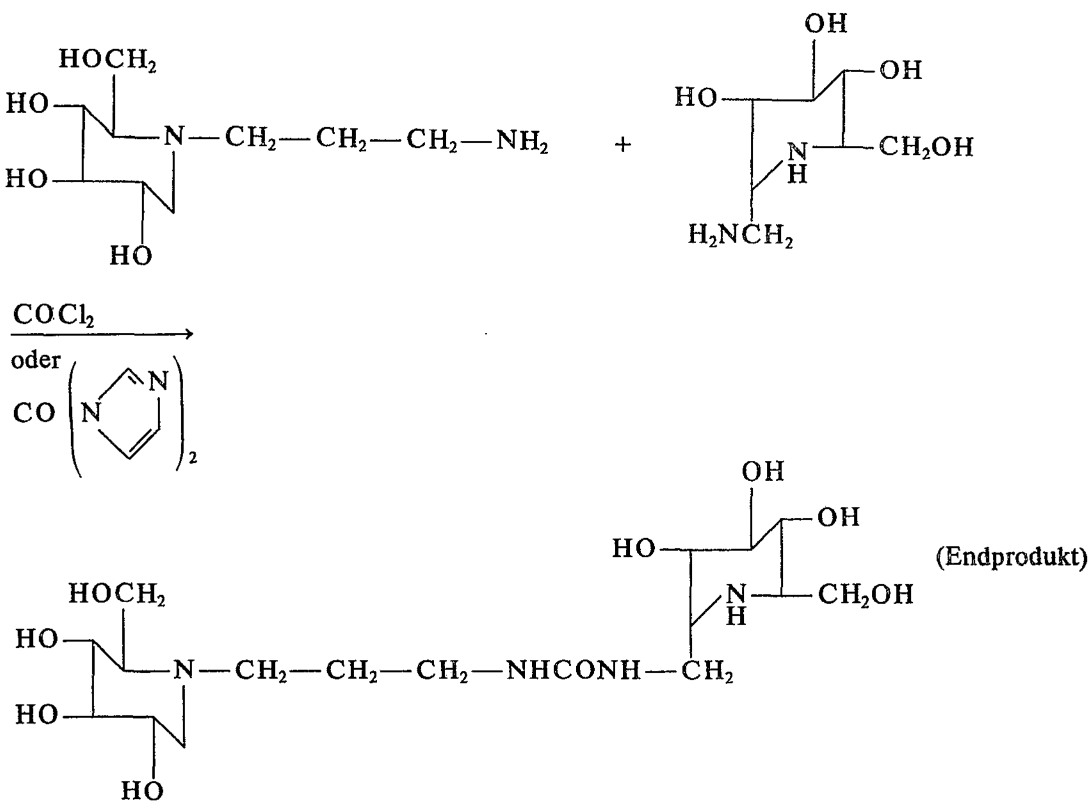


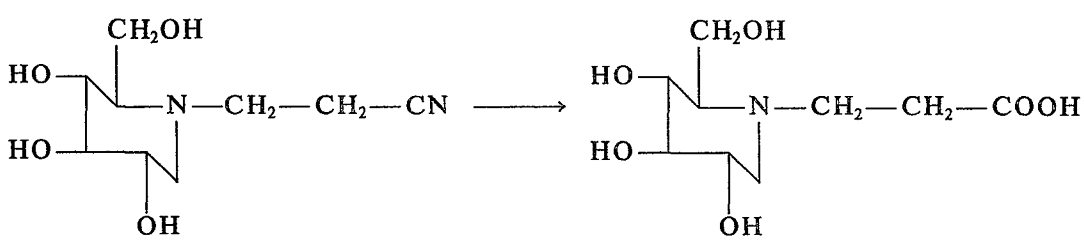

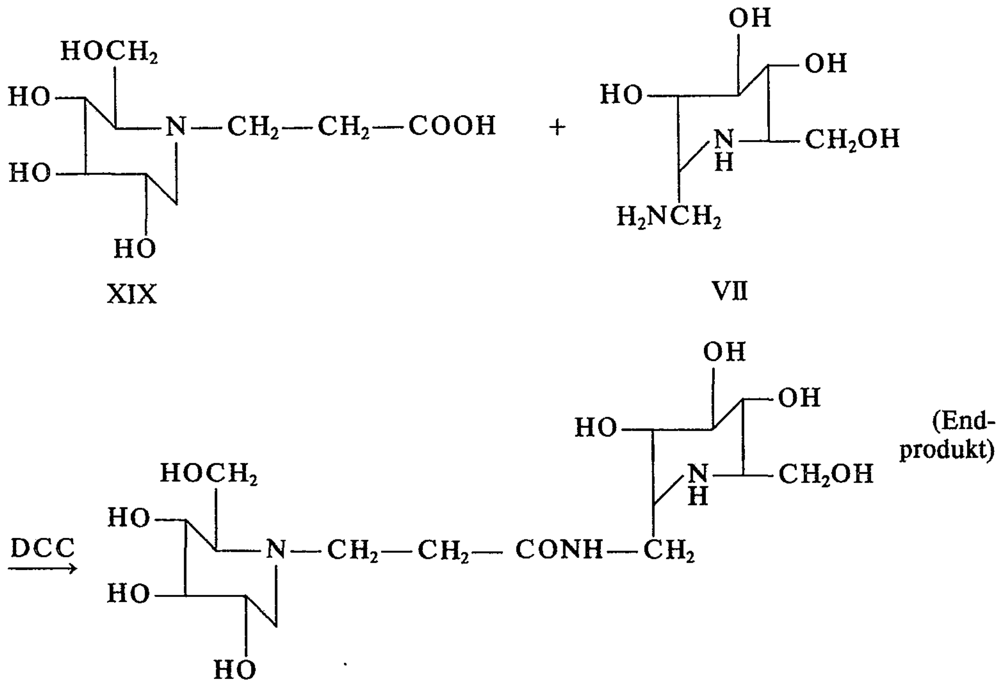


XIX + VII

Die Aminoverbindung kann dann mit Phosgen oder Carbonylbisimidazol und 1-Aminomethyl-1-desoxynojirmycin zu einer Verbindung der Formel I eingesetzt werden, die Carbonsäure kann mit Cyclohexylcarbodiimid und 1-Aminomethyl-1-desoxynjirmycin zu einer Verbindung der Formel I abreagieren.

Die als Ausgangsstoffe verwendeten Piperidinderivate 1-Desoxynojirimycin, 1-Aminomethyl-1-desoxynojirimycin und 1-Cyano-1-desoxynojirimycin sind aus EP-A 947 bekannt.

5-Aminomethyl-1-desoxynojirimycin wird durch katalytische Hydrierung (Raney-Nickel, 3,5 bar $H_2$, Lösungsmittel Wasser) von 2,6-Amino-2-hydroxymethyl-2,6-didesoxy-L-idohexonsäurenitril erhalten, das wiederum aus 6-Amino-6-desoxy-L-sorbofuranose-hydrochlorid-monohydrat (bekannt) und Natriumcyanid in 0,5 n Salzsäure bei Raumtemperatur hergestellt wird.

Ebenfalls bekannt oder nach bekannten Methoden herstellbar sind die zur N-Alkylierung dieser Piperidinderivate verwendeten Carbonylverbindungen, Alkylhalogenide und Sulfonsäureester.

Ebenfalls bekannt oder nach bekannten Methoden herstellbar sind die zur Umsetzung mit den Piperidinderivaten verwendeten reaktiven Säurederivate.

Für die reduktive Alkylierung kann man als Wasserstoffdonor katalytisch erregten Wasserstoff verwenden. Als Katalysator kommt vor allem Raney-Nickel in Frage, es können aber auch Edelmetallkatalysatoren Verwendung finden. Die Reaktion wird im allgemeinen bei Drucken zwischen 1 und 150 Atmosphären $H_2$-Druck und Temperaturen zwischen 20 und 150°C durchgeführt. Als Lösungsmittel werden protische, polare Lösungsmittel besonders Alkohole bevorzugt.

Als Wasserstoffdonor-Reduktionsmittel werden auch Alkalimetallcyanoborhydride, Dialkylaminoborane und Alkalimetallborhydride verwendet. Besonders bevorzugt in dieser Verfahrensvariante ist die Verwendung von Natriumcyanoborhydrid. Die Reaktion wird im allgemeinen bei Raumtemperatur durchgeführt. Es kann aber auch günstig sein, auf Rückflußtemperatur zu erhitzen.

Das Verfahren wird üblicherweise in einem inerten Lösungsmittel durchgeführt. Obwohl wasserfreie aprotische Lösungsmittel eingesetzt werden können (z. B. Tetrahydrofuran, wenn das Reduktionsmittel Morpholinoboran ist), wird gewöhnlich doch ein protisches Lösungsmittel verwendet. Als solches eignet sich besonders ein niederes Alkanol. Es kann aber auch Wasser oder ein wäßriges niedriges Alkanol (z. B. wäßriges Methanol oder Ethanol) oder andere wäßrige Lösungsmittelsysteme, wie z. B. wäßriges Dimethylformamid, wäßriges Hexamethylphosphorsäuretriamid, wäßriges Tetrahydrofuran oder wäßriger Ethylenglykoldimethylether verwendet werden.

Das Verfahren wird gewöhnlich in einem pH-Bereich von 1 bis 11 durchgeführt, bevorzugt ist ein pH-Bereich zwischen 4 und 7.

Die Umsetzung der Piperidinderivate mit Alkylhalogeniden wird in polaren, protischen oder aprotischen Lösungsmitteln zweckmäßigerweise in Gegenwart eines säurebindenden Mittels bei Temperaturen zwischen 0°C und Siedetemperatur des Lösungsmittels durchgeführt.

Bevorzugt wird die Reaktion in DMF/$H_2O$ mit $Ag_2O$ als säurebindendem Mittel oder in DMF mit $K_2CO_3$ als Säurefänger durchgeführt.

Die Umsetzung der Piperidinderivate mit den reaktiven Säurederivaten wird in polaren protischen oder aprotischen Lösungsmitteln durchgeführt. Gegebenenfalls wird ein säurebindendes Mittel zugesetzt. Die Reaktionstemperatur liegt zwischen −70°C und Siedetemperatur. Bevorzugtes Lösungsmittel für die Umsetzung mit Carbonsäurechloriden ist ein Gemisch aus Essigester, Methanol und $H_2O$ und als säurebindendes Mittel wird bevorzugt eine organische Base z. B. Triethylamin verwendet.

Ein Gemisch aus Essigester, Methanol und Wasser ist auch bevorzugtes Lösungsmittel für die Umsetzung der Piperidinderivate mit Isocyanaten, während für die Umsetzung mit Sulfonsäurechloriden bevorzugt DMF als Lösungsmittel und $K_2CO_3$ als säurebindendes Mittel verwendet werden.

Die erfindungsgemäßen Inhibitoren eignen sich als Therapeutica für folgende Indikationen:

Prädiabetes, Gastritis, Obstipation, Infektionen des Gastrointestinal-Traktes, Meteorismus, Flatulenz, Karies, Atherosklerose, Hypertension und besonders Adipositas, Diabetes und Hyperlipämie.

Zur Verbreiterung des Wirkungsspektrums kann es sich empfehlen, Inhibitoren für Glycosidhydrolasen, die sich gegenseitig in ihrer Wirkung ergänzen, zu kombinieren, sei es, daß es sich um Kombinationen der erfindungsgemäßen Inhibitoren untereinander oder um Kombinationen der erfindungsgemäßen Inhibitoren mit bereits bekannten handelt. So kann es beispielsweise zweckmäßig sein, erfindungsgemäße Saccharase-Inhibitoren mit bereits bekannten Amylase-Inhibitoren zu kombinieren.

Vorteilhaft sind in manchen Fällen auch Kombinationen der erfindungsgemäßen Inhibitoren mit bekannten oralen Antidiabetica ($\beta$-cytotrope Sulfonylharnstoffderivate und/oder blutzuckerwirksame Biguanide) sowie mit blutlipidsenkenden Wirkstoffen wie Clofibrat, Nicotinsäure, Cholestyramin und anderen.

Die Verbindungen können ohne Verdünnung, z. B. als Pulver oder in einer Gelatinehülle oder in Kombination mit einem Trägerstoff in einer pharmazeutischen Zusammensetzung appliziert werden.

Pharmazeutische Zubereitungen können eine größere oder kleinere Menge des Inhibitors enthalten, z. B. 0,1% bis 99,5%, in Kombination mit einem pharmazeutisch verträglichen nichttoxischen, inerten Trägerstoff, wobei der Trägerstoff eine oder mehrere feste, halbfeste oder flüssige Verdünnungsmittel, Füllstoffe und/oder nichttoxische inerte und pharmazeutisch-verträgliche Formulierungshilfsmittel enthalten kann. Solche pharmazeutischen Zubereitungen liegen vorzugsweise in Form von Dosierungseinheiten vor, d. h. physikalisch-diskreten, eine bestimmte Menge des Inhibitors enthaltenden Einheiten, die einem Bruchteil oder einem Vielfachen der Dosis entsprechen, die zur Herbeiführung der gewünschten Hemmwirkung erforderlich sind. Die Dosierungseinheiten können 1, 2, 3, 4 oder mehr Einzeldosen oder 1/2, 1/3 oder 1/4 einer Einzeldosis enthalten. Eine Einzeldosis enthält vorzugsweise eine genügende Menge Wirkstoff, um bei einer Applikation gemäß eines vorher bestimmten Dosierungsschemas einer oder mehrerer Dosierungseinheiten die gewünschte Hemmwirkung zu erzielen, wobei eine ganze, eine halbe, oder ein Drittel oder ein Viertel der Tagesdosis gewöhnlich zu allen, Haupt- und Nebenmahlzeiten am Tage verabreicht wird.

Andere therapeutische Mittel können auch eingenommen werden. Obgleich die Dosierung und das Dosierungsschema in jedem Fall sorgsam abgewogen werden sollte, unter Anwendung gründlichen fachmännischen Urteils und unter Beachtung des Alters, des Gewichtes und des Zustandes des Patienten, der Art und der Schwere der Erkrankung, wird die Dosierung gewöhnlich in einem Bereich zwischen etwa 1 bis etwa $1 \times 10^4$ SIE/kg des Körpergewichtes pro Tag liegen. In manchen Fällen wird man dabei eine ausreichende therapeutische Wirkung mit einer geringeren Dosis erreichen, während in anderen Fällen eine größere Dosis erforderlich sein wird.

Orale Applikation kann unter Verwendung fester und flüssiger Dosierungseinheiten durchgeführt werden, wie z. B. Pulver, Tabletten, Dragees, Kapseln, Granulate, Suspensionen, Lösungen und dergleichen.

Pulver wird durch Zerkleinerung der Substanz in einer geeigneten Größe und Vermischen mit einem ebenfalls zerkleinerten pharmazeutischen Trägerstoff hergestellt. Obgleich ein eßbares Kohlenhydrat, wie z. B. Stärke, Lactose, Saccharose oder Glucose normalerweise zu diesem Zwecke Verwendung findet und auch hier benutzt werden kann, ist es wünschenswert, ein nicht metabolisierbares Kohlenhydrat, wie z. B. ein Cellulosederivat, zu benutzen.

Süßmittel, Geschmackszusätze, Konservierungsstoffe, Dispergiermittel und Färbemittel können auch mitverwendet werden.

Die Kapseln können durch Zubereitung der oben beschriebenen Pulvermischung und durch Füllung bereits gebildeter Gelatinehüllen hergestellt werden. Die Pulvermischung kann man vor dem Füllvorgang mit Gleitmitteln, wie z. B. Kieselgel, Talkum, Magnesiumstearat, Calciumstearat oder festem Polyäthylenglykol versetzen. Die Mischung kann man ebenfalls mit einem Desintegrator oder Lösungsvermittler, wie z. B. Agar-Agar, Calciumcarbonat oder Natriumcarbonat, versetzen, um bei Einnahme der Kapsel die Zugänglichkeit des Inhibitors zu verbessern.

Die Anfertigung der Tabletten erfolgt z. B. durch Herstellung einer Pulvermischung, grob oder feinkörnig, und Hinzufügung eines Gleitmittels und Desintegrators. Aus dieser Mischung formt man Tabletten. Eine Pulvermischung bereitet man vor durch Mischung der Substanz, welche in geeigneter Weise zerkleinert wurde und ergänzt ein Verdünnungsmittel oder eine andere Trägersubstanz wie oben beschrieben. Gegebenenfalls fügt man ein Bindemittel hinzu: z. B. Carboxymethylcellulose, Alginate, Gelatine oder Polyvinylpyrrolidone, einen Lösungsverzögerer, wie z. B. Paraffin, einen Resorptionsbeschleuniger, wie z. B. ein quartärnares Salz und/oder ein Adsorptionsmittel, wie z. B. Bentonit, Kaolin oder Dicalciumphosphat. Die Pulvermischung kann granuliert werden zusammen mit einem Bindemittel, wie z. B.

Sirup, Stärkepaste, Akazienschleim, oder Lösungen aus Zellulose- oder Polymerenmaterialien. Danach preßt man das Produkt durch ein grobes Sieb. Als Alternative hierzu kann man die Pulvermischung durch eine Tablettenmaschine laufen lassen und die sich ergebenden ungleichmäßig geformten Stücke bis auf Korngröße zerkleinern. Damit die entstandenen Körner nicht in den tablettenbildenden Düsen stecken bleiben, kann man sie mit einem Gleitmittel versetzen, wie z. B. Stearinsäure, Stearatsalz, Talkum oder Mineralöl. Diese gleitfähig gemachte Mischung wird dann in Tablettenform gepreßt. Die Wirkstoffe können auch mit freifließenden inerten Trägerstoffen vereinigt werden und direkt in Tablettenform gebracht werden unter Auslassung der Granulat- oder Zerstückelungsschritte. Man kann das Produkt mit einer klaren oder opaken Schutzhülle versehen, z. B. einem Überzug aus Schellack, einem Überzug aus Zucker oder Polymersubstanzen und einer polierten Hülle aus Wachs. Farbstoffe können diesen Überzügen beigefügt werden, damit zwischen den verschiedenen Dosierungseinheiten unterschieden werden kann.

Die oral zu verabreichenden Zubereitungsformen, wie z. B. Lösungen, Syrup und Elixire, lassen sich in Dosierungseinheiten herstellen, so daß eine bestimmte Menge Präparat eine bestimmte Menge Wirkstoff enthält. Syrup kann so hergestellt werden, daß der Wirkstoff in einer wäßrigen Lösung, welche geeignete Geschmacksstoffe enthält, gelöst wird; Elixire werden unter Verwendung nichttoxischer, alkoholischer Trägerstoffe erhalten. Suspensionen kann man durch Dispergieren der Verbindung in einem nichttoxischen Trägerstoff darstellen. Lösungsvermittler und Emulgiermittel, wie z. B. äthoxylierte Isostearylalkohole und Polyoxyäthylensorbitester, Konservierungsmittel, geschmacksverbessernde Zusätze wie z. B. Pfefferminzöl oder Saccharin und dergleichen können auch zugegeben werden.

Dosierungsvorschriften können auf der Kapsel angegeben werden. Überdies kann die Dosierung so abgesichert sein, daß der Wirkstoff verzögert abgegeben wird, z. B. durch Einhalten des Wirkstoffes in Polymerensubstanzen, Wachse oder dergleichen.

Zusätzlich zu den oben erwähnten pharmazeutischen Zusammensetzungen lassen sich auch diese Wirkstoffe enthaltende Lebensmittel herstellen; beispielsweise Zucker, Brot, Kartoffelprodukte, Fruchtsaft, Bier, Schokolade und andere Konfektartikel, und Konserven, wie z. B. Marmelade, wobei zu diesen Produkten eine therapeutisch-wirksame Menge mindestens eines der erfindungsgemäßen Inhibitoren gegeben wurde.

Die unter Verwendung der erfindungsgemäßen Wirkstoffe hergestellten Nahrungsmittel eignen sich sowohl zur Diät bei Patienten, die an Stoffwechselstörungen leiden als auch zur Ernährung gesunder Personen im Sinne einer Stoffwechselstörungen vorbeugenden Ernährungsweise.

Die erfindungsgemäßen Inhibitoren weisen weiterhin die Eigenschaft auf, in Tieren das Verhältnis des Anteils an unerwünschtem Fett zum Anteil des erwünschten fettarmen Fleisches (mageres Fleisch) zugunsten des mageren Fleisches in hohem Maße zu beeinflussen. Dies ist von besonderer Bedeutung für die Aufzucht und Haltung von landwirtschaftlichen Nutztieren, z. B. in der Schweinemast, aber auch von erheblicher Bedeutung für die Aufzucht und Haltung von sonstigen Nutztieren und Ziertieren. Die Verwendung der Inhibitoren kann weiterhin zu einer erheblichen Rationalisierung der Fütterung der Tiere führen, sowohl zeitlich, mengenmäßig wie auch qualitätsmäßig. Da sie eine gewisse Verzögerung der Verdauung bewirken, wird die Verweildauer der Nährstoffe im Verdauungstrakt verlängert, wodurch eine mit weniger Aufwand verbundene ad libitum-Fütterung ermöglicht wird. Weiterhin ergibt sich bei der Verwendung der erfindungsgemäßen Inhibitoren in vielen Fällen eine erhebliche Einsparung von wertvollem Proteinfutter.

Die Wirkstoffe können somit praktisch in allen Bereichen der Tierernährung als Mittel zur Reduzierung des Fettansatzes sowie der Einsparung von Futtereiweiß verwendet werden.

Die Wirksamkeit der Wirkstoffe ist hierbei weitgehend unabhängig von der Art und dem Geschlecht der Tiere. Besonders wertvoll erweisen sich die Wirkstoffe bei Tierarten, die überhaupt oder in bestimmten Lebensabschnitten zu stärkerer Fetteinlagerung neigen.

Als Tiere, bei denen die Inhibitoren zur Reduzierung des Fettansatzes und/oder zur Einsparung von Futtereiweiß eingesetzt werden können, seien beispielsweise folgende Nutz- und Ziertiere genannt: Warmblüter wie Rinder, Schweine, Pferde, Schafe, Ziegen, Katzen, Hunde, Kaninchen, Pelztiere, z. B. Nerze, Chinchilla, andere Ziertiere, z. B. Meerschweinchen und Hamster, Labor- und Zootiere, z. B. Ratten, Mäuse, Affen usw.; Geflügel, z. B. Broiler, Hühner, Gänse, Enten, Truthähne, Tauben, Papageien und Kanarienvögel und Kaltblüter, wie Fische, z. B. Karpfen und Reptilien, z. B. Schlangen.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,1 mg bis 1,0 g, insbesondere 1 bis 100 mg/kg Futter pro Tag. Die Dauer der Verabreichung kann von wenigen Stunden oder Tagen bis zu mehreren Jahren betragen. Die passende Menge Wirkstoff sowie die passende Dauer der Verabreichung stehen in engem Zusammenhang mit dem Fütterungsziel. Sie hängen insbesondere von der Art, dem Alter, dem Geschlecht, dem Gesundheitszustand und der Art der Haltung der Tiere ab und sind durch jeden Fachmann leicht zu ermitteln.

Die erfindungsgemäßen Wirkstoffe werden den Tieren nach den üblichen Methoden verabreicht. Die Art der Verabreichung hängt insbesondere von der Art, dem Verhalten und dem Allgemeinzustand der Tiere ab. So kann die Verabreichung einmal oder mehrmals täglich, in regelmäßigen oder unregelmäßigen Abständen, oral erfolgen. Aus Zweckmäßigkeitsgründen ist in den meisten Fällen eine orale Verab-

reichung, insbesondere im Rhythmus der Nahrungs- und/oder Getränkeaufnahme der Tiere, vorzuziehen.

Die Wirkstoffe können als reine Stoffe oder in formulierter Form verabreicht werden, wobei die formulierte Form sowohl als Premix, also in Mischung mit nichttoxischen inerten Trägerstoffen beliebiger Art, als auch als Teil einer Gesamtration in Form eines Beifutters bzw. als Mischungsbestandteil eines alleinigen Mischfutters zu verstehen ist. Mit eingeschlossen ist auch die Applikation geeigneter Zubereitungen über das Trinkwasser.

Die Wirkstoffe können gegebenenfalls in formulierter Form auch zusammen mit anderen Nähr- und Wirkstoffen, z. B. Mineralsalzen, Spurenelementen, Vitaminen, Eiweißstoffen, Energieträgern (z. B. Stärke, Zucker, Fette), Farbstoffen und/oder Geschmacksstoffen oder anderen Futterzusatzstoffen, wie z. B. Wachstumsförderern, in geeigneter Form verabreicht werden. Die Wirkstoffe können den Tieren vor, während oder nach der Nahrungsaufnahme gegeben werden.

Empfehlenswert ist die orale Verabreichung zusammen mit dem Futter und/oder Trinkwasser, wobei je nach Bedarf die Wirkstoffe der Gesamtmenge oder nur Teilen des Futters und/oder Trinkwassers zugegeben werden.

Die Wirkstoffe können nach üblichen Methoden durch einfaches Mischen als reine Stoffe, vorzugsweise in fein verteilter Form oder in formulierter Form in Mischung mit eßbaren, nichttoxischen Trägerstoffen, gegebenenfalls auch in Form eines Premix oder eines Futterkonzentrates, dem Futter und/oder dem Trinkwasser beigefügt werden.

Das Futter und/oder Trinkwasser kann beispielsweise die erfindungsgemäßen Wirkstoffe in einer Konzentration von etwa 0,001 bis 5,0%, insbesondere 0,02 bis 2,0% (Gewicht) enthalten. Die optimale Höhe der Konzentration des Wirkstoffs im Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen, handelsüblichen oder speziellen Futterzusammensetzungen verwendet werden, die vorzugsweise das übliche, für eine ausgewogene Ernährung notwendige Gleichgewicht aus Energie- und Eiweißstoffen, einschließlich Vitaminen und Mineralstoffen enthalten. Das Futter kann sich beispielsweise zusammensetzen aus pflanzlichen Stoffen, z. B. Ölkuchenschroten, Getreideschroten, Getreidenebenprodukten, aber auch aus Heu, Gärfutter, Rüben und anderen Futterpflanzen, aus tierischen Stoffen, z. B. Fleisch- und Fischprodukte, Knochenmehl, Fette, Vitamine, z. B. A, D, E, K und B-Komplex sowie spezielle Proteinquellen, z. B. Hefen sowie bestimmte Aminosäuren und Mineralstoffen und Spurenelementen, wie z. B. Phosphor und Eisen, Zink, Mangan, Kupfer, Kobalt, Jod.

Premixe können vorzugsweise etwa 0,1 bis 50%, insbesondere 0,5 bis 5,0% (Gewicht) eines erfindungsgemäßen Wirkstoffs neben beliebigen eßbaren Trägerstoffen und/oder Mineralsalzen, z. B. kohlensaurem Futterkalk enthalten und werden nach den üblichen Mischmethoden hergestellt.

Mischfutter enthaltend vorzugsweise 0,001 bis 5,0%, insbesondere 0,02 bis 2,0% (Gewicht) eines erfindungsgemäßen Wirkstoffs neben den üblichen Rohstoffkomponenten eines Mischfutters, z. B. Getreideschrote oder -nebenprodukte, Ölkuchenschrote, tierisches Eiweiß, Mineralien, Spurenelemente und Vitamine. Sie können nach den üblichen Mischmethoden hergestellt werden.

Vorzugsweise in Premixen und Mischfuttermitteln können die Wirkstoffe gegebenenfalls auch durch ihre Oberfläche bedeckenden geeigneten Mittel, z. B. mit nichttoxischen Wachsen oder Gelatine vor Luft, Licht und/oder Feuchtigkeit geschützt werden.

Beispiel für die Zusammensetzung eines fertigen Mischfutters für Geflügel, das einen erfindungsgemäßen Wirkstoff enthält:

200 g Weizen, 340 g Mais, 360,3 g Sojaschrot, 60 g Rindertalg, 15 g Dicalciumphosphat, 10 g Calciumcarbonat, 4 g jodiertes Kochsalz, 7,5 g Vitamin-Mineral-Mischung und 3,2 g Wirkstoff-Premix ergeben nach sorgfältigem Mischen 1 kg Futter.

Die Vitamin-Mineral-Mischung besteht aus:

6000 I. E. Vitamin A, 1000 I. E. Vitamin $D_3$, 10 mg Vitamin E, 1 mg Vitamin $K_3$, 3 mg Riboflavin, 2 mg Pyridoxin, 20 mg Vitamin $B_{12}$, 5 mg Calciumpantothenat, 30 mg Nikotinsäure, 200 mg Cholinchlorid, 200 g Mn $SO_4 \times H_2O$, 140 mg Zn $SO_4 \times 7\,H_2O$, 100 mg Fe $SO_4 \times 7\,H_2O$ und 20 mg Cu $SO_4 \times 5\,H_2O$.

Der Wirkstoff-Premix enthält einen erfindungsgemäßen Wirkstoff in der gewünschten Menge, z. B. 1600 mg und zusätzlich 1 g DL-Methionin sowie so viel Sojabohnenmehl, daß 3,2 g Premix entstehen.

Beispiel für die Zusammensetzung eines Schweinemischfutters, das einen Wirkstoff der Formel I enthält:

630 g Futtergetreideschrot (zusammengesetzt aus 200 g Mais-, 150 g Gerste-, 150 g Hafer- und 130 g Weizenschrot), 80 g Fischmehl, 60 g Sojaschrot, 58,8 g Tapiokamehl, 38 g Bierhefe, 50 g Vitamin-Mineral-Mischung für Schweine (Zusammensetzung, z. B. wie beim Kükenfutter), 30 g Leinkuchenmehl, 30 g Maiskleberfutter, 10 g Sojaöl, 10 g Zuckerrohrmelasse und 2 g Wirkstoff-Premix (Zusammensetzung z. B. beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

Die Inhibitoren können einzeln oder aber auch in beliebigen Mischungen untereinander verwendet werden.

Saccharase-Inhibitionstest in vitro

Der Saccharase-Inhibitionstest in vitro ermöglicht die Bestimmung der enzyminhibitorischen Aktivität einer Substanz durch den Vergleich der Aktivität des solubilisierten intestinalen Disaccharidasen-Komplexes in Gegenwart bzw. in Abwesenheit (sog. 100%-Wert) des Inhibitors. Als Substrat, welches die Spezifität des Inhibitionstestes bestimmt, dient dabei eine praktisch Glucosefreie Saccharose (Glucose $<$ 100 ppm); die Enzymaktivitätsbestimmung basiert auf der spektrophotometrischen Bestimmung freigesetzter Glucose mittels Glucose-Dehydrogenase und Nicotinamid-adenin-dinucleotid als Cofaktor.

Eine Saccharase-Inhibitor-Einheit (SIE) ist definiert als diejenige inhibitorische Aktivität, welche in einem definierten Testansatz eine vorgegebene saccharolytische Aktivität um eine Einheit (Saccharase-Einheit = SE) reduziert; die Saccharase-Einheit ist dabei als diejenige Enzymaktivität definiert, welche unter vorgegebenen Bedingungen ein $\mu$mol Saccharose pro Minute spaltet und damit zur Freisetzung von je ein $\mu$mol Glucose, welche im Test bestimmt wird, und Fructose, welche im Test nicht erfaßt wird, führt.

Der intestinale Disaccharidasen-Komplex wird aus Schweinedünndarm-Mucosa durch tryptische Verdauung, Fällung aus 66% Äthanol bei −20°C, Aufnehmen des Präcipitates in 100 mM Phosphat-Puffer, pH 7,0 und abschließende Dialyse gegen denselben Puffer gewonnen.

10 $\mu$l einer Probelösung, die so angesetzt ist, daß die Extinktion des Testansatzes mindestens 10%, jedoch nicht mehr als 25% unter der des 100%-Wertes liegt, werden mit 100 $\mu$l einer Verdünnung des intestinalen Disaccharidasen-Komplexes in 0,1 M Maleinat-Puffer, pH 6,25, versetzt und für 10 Minuten bei 37°C vorinkubiert. Die Verdünnung des Disaccharidasen-Komplexes ist auf eine Aktivität von 0,1 SE/ml einzustellen.

Anschließend wird die saccharolytische Reaktion durch Zugabe von 100 $\mu$l einer 0,4 M Lösung von Saccharose (»SERVA 35 579«) in 0,1 M Maleinat-Puffer, pH 6,25 gestartet und nach einer Inkubationsdauer von 20 Minuten bei 37°C durch die Zugabe von 1 ml Glucose-Dehydrogenase-Reagenz (1 Fläschchen Glucose-Dehydrogenase-Mutarotase-Gemisch lyophiliert (»MERCK 14 053«) und 331,7 mg $\beta$-Nicotinamid-adenin-dinucleotid (freie Säure, »BOEHRINGER« Reinheitsgrad I) in 250 ml 0,5 M Tris-Puffer, pH 7,6 gelöst) abgestoppt. Zum Nachweis der Glucose wird 30 Minuten bei 37°C inkubiert und schließlich bei 340 nm gegen einen Reagenzienblank (mit Enzym, jedoch ohne Saccharose) photometriert.

Die Berechnung der Hemmaktivität von Inhibitoren ist dadurch erschwert, daß schon geringfügige Änderungen im Testsystem, beispielsweise ein geringfügig von Bestimmung zu Bestimmung variierender 100%-Wert, von nicht mehr zu vernachlässigendem Einfluß auf das Testergebnis sind. Man umgeht diese Schwierigkeiten, indem man bei jeder Bestimmung einen Standard mitlaufen läßt; als Standard dient ein Saccharose-Inhibitor der Formel $C_{25}H_{43}O_{18}N$, welcher eine spezifische Hemmaktivität von 77 700 SIE/g aufweist und bei eingesetzten Mengen von 10 bis 20 ng im Test zu einer Hemmung von oben spezifizierter Größenordnung führt. Bei Kenntnis der Differenz der Extinktionen bei 340 nm von 100%-Wert und durch Standard gehemmtem Ansatz läßt sich aus der Extinktionsdifferenz von 100%-Wert und durch die Probelösung gehemmtem Ansatz unter Berücksichtigung der eingesetzten Menge an Inhibitor in bekannter Weise dessen spezifische Hemmaktivität errechnen, ausgedrückt in Saccharase-Inhibitor-Einheiten pro Gramm (SIE/g).

Beispiel 1

1,5-Bis-[N,N'(-1,5-Imino-1,5-didesoxy-D-glucityl)]-pentan

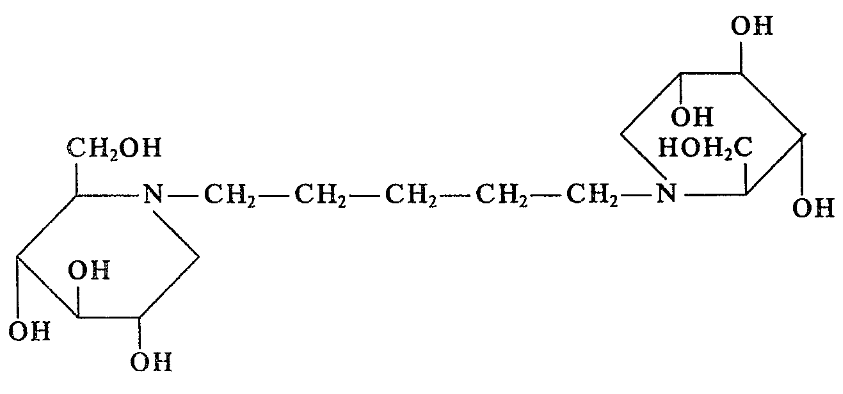

Eine Lösung von 21 g 1-Desoxynojirimycin in 630 ml Methanol und 42,5 ml Eisessig wird mit 28,4 ml 25%igem Pentandial versetzt und auf ca. −5°C abgekühlt. Dann werden 17,5 g Natriumcyanoborhydrid zugefügt. Nach vierstündigem Rühren bei 0°C wird 18 Stunden bei Raumtemperatur gerührt. Es wird eingeengt, in ca. 200 ml Methanol/Wasser in Volumenverhältnis 8 : 1 gelöst und auf eine 40 cm lange und 6 cm weite Säule aufgetragen, die als Füllmittel Amberlite IR 120 ($H^+$-Form) enthält. Es wird mit ca. 4 Liter Methanol/Wasser 8 : 1 gewaschen und anschließend mit 5% Ammoniak in Methanol/Wasser 8 : 1 eluiert. Man erhält ein Gemisch von drei Substanzen, von denen eine 1-Desoxynojirimycin ist. Die gesammelten Fraktionen werden eingeengt.

Man erhält 20,6 g eines leicht gefärbten Öles. Dieses Gemisch wird als konzentrierte wäßrige Lösung auf eine 120 cm lange und 6 cm weite Säule aufgetragen, die als stationäre Phase Zellulose und als mobile Phase Aceton enthält. Es wird mit Aceton und anschließend mit wäßrigem Aceton eluiert, wobei der Anteil des Wassers kontinuierlich vergrößert wird. Die Überprüfung der einzelnen Fraktionen erfolgt durch Dünnschichtchromatographie. Nachdem die Nebenprodukte abgetrennt waren, wird das Produkt mit Aceton/Wasser im Volumenverhältnis 3 : 1 erhalten. Die sauberen Fraktionen werden gesammelt und eingeengt. Der Eindampfrückstand wird in absolutem Methanol gelöst, nach Zugabe von Tonsil und Tierkohle filtriert und eingeengt. Der Eindampfrückstand wird in wenig absolutem Methanol aufgenommen und kristallisieren gelassen. Nach 18stündigem Stehen wird abgesaugt und mit Methanol gewaschen. Man erhält 10,1 g farblose Kristalle von Schmelzpunkt 186–187°C.

Beispiel 2

N,N'-Bis-[1,5-Imino-1,5-didesoxy-D-glucityl)-methyl]-3,3'-sulfonyl-bisbenzolsulfonsäureamid

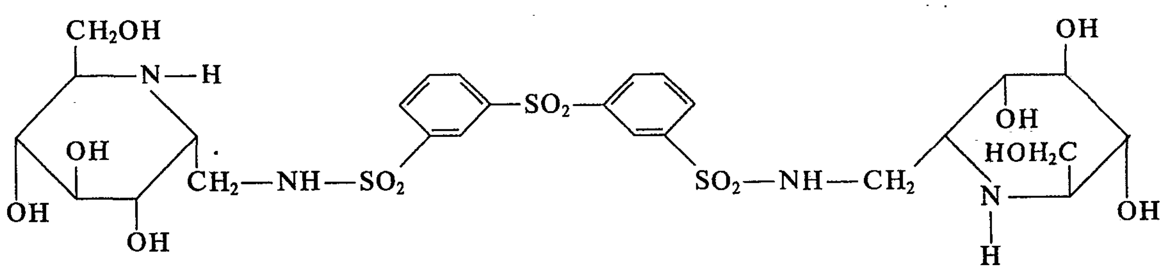

Eine Suspension von 1,92 g 1-Aminomethyl-1-desoxynojirimycin und 2,76 g Kaliumcarbonat in 20 ml absolutem DMF wird auf −10°C abgekühlt und mit 1,85 g 3,3-Sulfonylbisbenzolsulfonylchlorid versetzt. Es wird über Nacht gerührt, vom abgeschiedenen Salz abgesaugt und eingeengt. Der Eindampfrückstand wird in wenig Wasser gelöst und auf eine 120 cm lange und 4 cm weite Säule aufgetragen, die als stationäre Phase Zelulose und als mobile Phase Aceton enthält. Es wird mit wäßrigem Aceton mit steigendem Wassergehalt eluiert. Die einzelnen Fraktionen werden dünnschichtchromatographisch überprüft. Die Fraktionen mit dem gewünschten Produkt werden zusammengefaßt und eingeengt.

Man erhält 0,9 g eines farblosen Schaumes mit einem Rf-Wert von 0,57 (DC-Fertigplatten, Merck mit Kieselgel 60 F 254, Fließmittel: Methanol/Chloroform/25%iger Ammoniak 90 : 60 : 60). Rf-Wert von 1-Aminomethyl-1-desoxynojirimycin = 0,26.

Beispiel 3

$N^1$,$N^4$-Bis-[5-(1,5-Imino-1,5-didesoxy-D-glucityl)-methyl]-hexamethylen-bisharnstoff

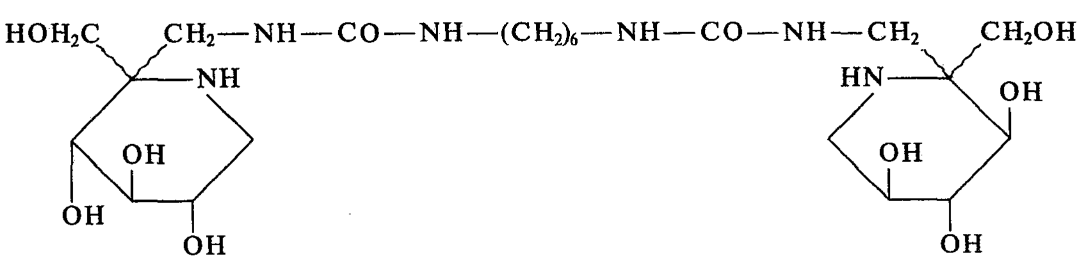

5,3 g 2-Aminomethyl-2-hydroxymethyl-3,4,5-trihydroxypiperidin-dihydrochlorid werden in 40 ml 1 n Natronlauge gelöst und mit 80 m Methanol verdünnt. Es wird auf 0°C abgekühlt und tropfenweise mit einer Lösung von 1,59 ml Hexamethylendiisocyanat in 20 ml Essigester versetzt. Es wird 18 Stunden gerührt, eingeengt, in absolutem Methanol aufgenommen und vom anorganischen Salz filtriert. Das Filtrat wird konzentriert und auf eine 120 cm lange und 4 cm weite Säule aufgetragen, die als stationäre Phase Zellulose und als mobile Phase Aceton enthält. Es wird mit wäßrigem Aceton eluiert, wobei die Konzentration an Wasser schrittweise erhöht wird. Die einzelnen Fraktionen werden dünnschichtchromatographisch auf ihren Gehalt an der gewünschten Verbindung hin überprüft.

Die Fraktionen mit dem gewünschten Produkt werden zusammengefaßt und eingeengt. Man erhält 450 mg in Form eines farblosen Schaumes mit einem Rf-Wert = 0,33, Fließmittel: Methanol/Chloroform/25%iger-Ammoniak 3 : 2 : 2. Rf-Wert von 2-Aminomethyl-2-hydroxymethyl-3,4,5-trihydroxypiperidin = 0,315.

## Beispiel 4

N,N'-Bis-[5-(1,5-Imino-1,5-didesoxy-D-glucityl)-methyl]-sebacinsäurediamid

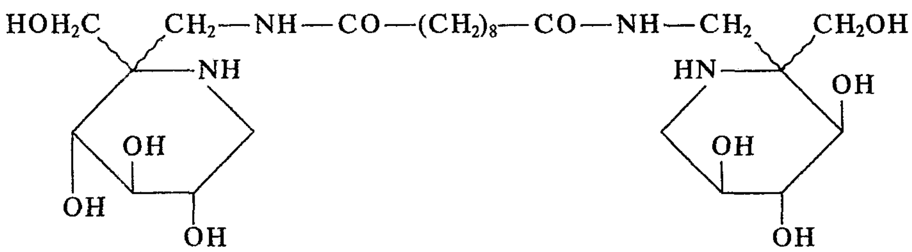

5,3 g 2-Aminomethyl-2-hydroxymethyl-3,4,5-trihydroxypiperidin-dihydrochlorid werden in 20 ml Wasser und 60 ml Methanol gelöst und mit 8,4 ml Triethylamin versetzt. Unter Eiskühlung wird dann eine Lösung von 2,13 ml Sebacinsäuredichlorid in 30 ml Essigester zugetropft. Es wird 3 Stunden bei Raumtemperatur gerührt, eingeengt und als wäßrige Lösung durch eine 25 cm lange und 3 cm weite Säule filtriert, die als Füllmittel Amberlite IR 400 ($OH^{\ominus}$-Form) enthält. Es wird gut mit Wasser gewaschen und eingeengt. Der Eindampfrückstand wird auf eine 120 cm lange und 4 cm weite Säule aufgetragen, die als stationäre Phase Zellulose und als mobile Phase Aceton enthält. Es wird zuerst mit Aceton und dann mit Aceton, dem schrittweise größere Anteile an Wasser zugefügt werden, eluiert. Die einzelnen Fraktionen werden dünnschichtchromatographisch untersucht. Die Fraktionen mit dem gewünschten Produkt werden zusammengefaßt und eingeengt. Man erhält 0,9 g der Verbindung als festen Schaum mit einem Rf-Wert von 0,48 (Fließmittel: Methanol/Chloroform/25%iger Ammoniak 3 : 2 : 2).

## Beispiel 5

N,N'-Bis-[1-$\alpha$-(1,5-didesoxy-1,5-imino-D-glucityl)methyl]-sebacinsäurediamid

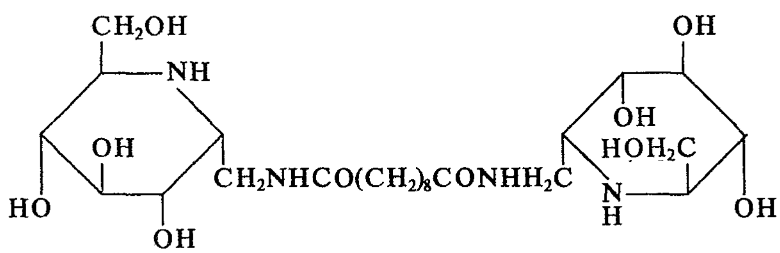

Zu 0,5 g 1-Aminomethyl-1-desoxynojirimycin in 6 ml Methanol, 2 ml Wasser und 0,36 ml Triethylamin tropft man bei 0°C 0,28 ml Sebacinsäuredichlorid in 4 ml Essigester. Man rührt 2 Stunden bei Raumtemperatur nach und saugt den Niederschlag ab. Man wäscht mit Essigester nach und trocknet.

Der Feststoff wird zur Reinigung in DMF/$H_2O$ gelöst und mit Aceton gefällt.

Ausbeute: 0,5 g. F. P.: 247—250°C. Rf-Wert auf DC-Fertigplatten (Merck mit Kieselgel 60 F 254) mit Methanol/Chloroform/wäßrigem Ammoniak 3 : 2 : 2 als Fließmittel: 0,53. Rf-Wert für 1-Aminomethyl-1-desoxynojirimycin: 0,28.

Beispiel 6

1,4-[N,N'-Bis-(1',5'-didesoxy-1',5'-imino-D-glucit)-yl]-buten-2

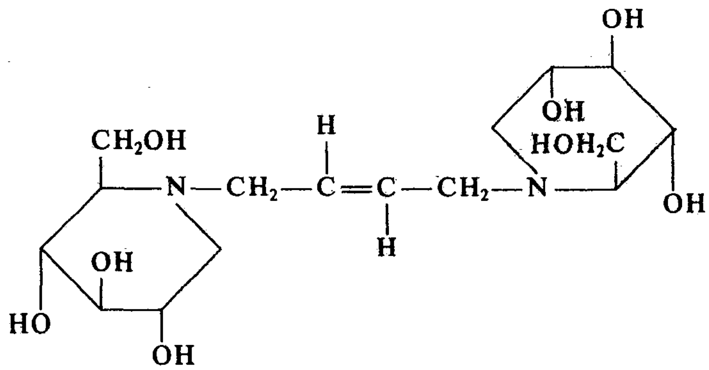

Zu 2 g 1-Desoxynojirimycin in 12 ml $H_2O$ und 12 ml DMF gibt man 2 g $Ag_2O$ und tropft bei 0°C 1,3 g trans-1,4-Dibrombuten-2 in 5 ml DMF hinzu.

Man läßt langsam auf Raumtemperatur kommen und rührt 3 Stunden bei Raumtemperatur. Anschließend wird mit 30 ml Wasser verdünnt, die Salze werden abfiltriert und das wäßrige Filtrat wird eingeengt. Der Rückstand wird auf eine mit Zellulose gefüllte Säule aufgetragen.

Es wird zunächst mit Aceton/Wasser 9 : 1, dann mit Aceton/Wasser 8 : 2 und schließlich mit Aceton/Wasser 7 : 3 eluiert.

Die Fraktionen werden dünnschichtchromatographisch auf Kieselgelplatten geprüft (Fließmittel: Methanol/Chloroform/wäßriges Ammoniak 3 : 2 : 2).

Die Fraktionen, die die gesuchte Verbindung enthalten, werden zusammengefaßt und eingeengt.

Ausbeute: 0,9 g. Rf-Wert auf DC-Fertigplatten (Merck, Darmstadt, Kieselgel 60 F 254) mit Methanol/Chloroform/wäßrigem Ammoniak 3 : 2 : 2 als Fließmittel: 0,35. Rf-Wert für 1-Desoxynojirimycin: 0,51.

Beispiel 7

Analog Beispiel 3 wurde erhalten:

N[1],N[4]-Bis[1-$\alpha$-(1,5-didesoxy-1,5-imino-D-glucityl)-methyl]-hexamethylen-bisharnstoff

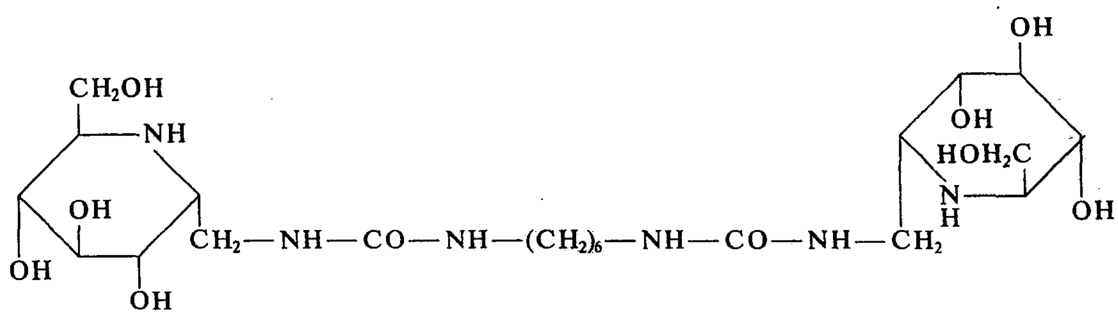

Rf-Wert: 0,39, 1-Aminomethyl-1-desoxynojirimycin: 0,28.

Beispiel 8

*β,β'*-[N,N'-Bis-(1,5-didesoxy-1,5-imino-D-glucit)-yl]-diethylsulfon

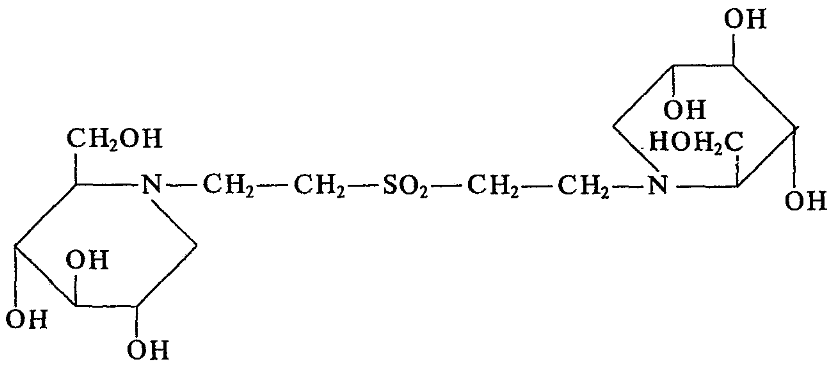

6,52 g (40 m Mol) 1-Desoxynojirimycin werden in einem Gemisch von 60 ml Ethanol und 30 ml Wasser gelöst und nach Zugabe eines Tropfens konzentrierter Natronlauge auf 0—5°C abgekühlt. Es wird mit 2,3 ml (~22 m Mol) Divinylsulfon versetzt und anschließend 48 Stunden bei Raumtemperatur gerührt. Es wird eingeengt, in wenig Wasser gelöst und auf eine 120 cm lange und 6 cm weite Säule aufgetragen, die als stationäre Phase Zellulose und als mobile Phase Aceton enthält. Es wird mit Aceton eluiert, dem stufenweise ein immer größerer Anteil Wasser zugefügt wird. Die einzelnen Fraktionen werden dünnschichtchromatographisch überprüft. Nach Abtrennen der Nebenprodukte erhält man die gewünschte Verbindung durch Eluation mit Aceton/Wasser im Volumenverhältnis 7 : 3. Die Fraktionen mit dem reinen Produkt werden gesammelt und eingeengt. Man erhält 4,6 g eines farblosen Schaumes mit einem Rf-Wert von 0,33 (Fließmittel: Methanol/Chloroform/25%iger Ammoniak 3 : 2 : 2), Rf-Wert für 1-Desoxynojirimycin = 0,52.

Beispiel 9

$N^1$,$N^4$-Bis-[5-(1,5-Imino-1,5-didesoxy-D-glucityl)-methyl]-hexamethylen-bisthioharnstoff

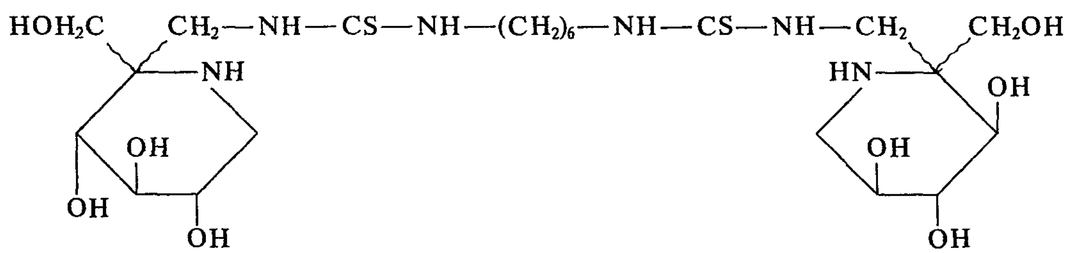

5,3 g 2-Aminomethyl-2-hydroxymethyl-3,4,5-trihydroxy-piperidin-dihydrochlorid werden in einem Gemisch von 180 ml Methanol und 60 ml Wasser gelöst und nach Zugabe von 5,6 ml Triethylamin tropfenweise bei 0 bis —5°C mit einer Lösung von 2,0 g Hexamethylendiisothiocyanat in 60 ml Essigester versetzt. Es wird 24 Stunden bei Raumtemperatur gerührt, eingeengt und in wenig Methanol/Wasser im Volumenverhältnis von 8 : 1 gelöst. Es wird durch eine kurze Säule filtriert, die Amberlite IR 400 OH-Form enthält, und gut mit Methanol/Wasser 8 : 1 gewaschen. Das Filtrat wird eingeengt, in wenig Methanol/Wasser gelöst und auf eine 100 cm lange und 4 cm weite Säule gegeben, die als stationäre Phase Zellulose und als mobile Phase Aceton enthält. Es wird zunächst mit Aceton/Wasser 9 : 1, dann mit Aceton/Wasser 8,5 : 1,5 und schließlich mit Aceton/Wasser 8 : 2 eluiert. Die Fraktionen werden dünnschichtchromatographisch auf Kieselgelplatten überprüft (Fließmittel: Methanol/Chloroform/25%iger Ammoniak 3 : 2 : 2). Die Fraktionen mit dem reinen Produkt werden zusammengefaßt und eingeengt. Man erhält 1,4 g der Verbindung 9 mit einem Rf-Wert von 0,56.

Rf-Wert von 2-Aminomethyl-2-hydroxymethyl-3,4,5-trihydroxy-piperidin-dihydrochlorid = 0,34. Fließmittel: Methanol/Chloroform/25%iger Ammoniak im Volumenverhältnis 3 : 2 : 2.

Beispiel 10

1,6-[N,N'-Bis-(1',5'-didesoxy-1',5'-imino-D-glucit)-yl]-n-hexan

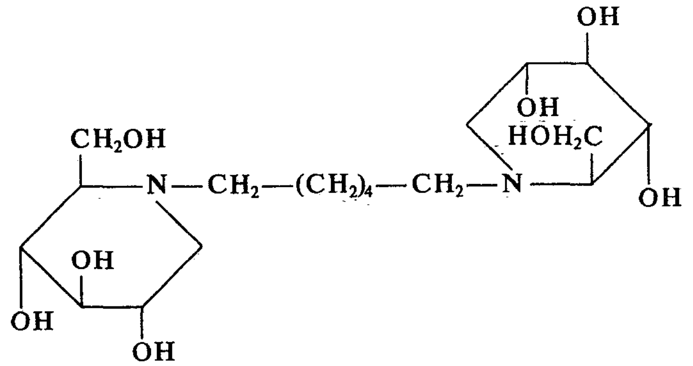

Zu 5 g 1-Desoxynojirimycin in 50 ml DMF gibt man bei 20°C 2,5 g $K_2CO_3$ und 4 g 1,6 Dibromhexan. Man erwärmt auf 100°C und läßt 15 Stunden rühren. Anschließend wird das DMF im Vakuum abgezogen. Der Rückstand wird auf eine mit Zellulose gefüllte Säule aufgetragen. Es wird zunächst mit Aceton/Wasser 9 : 1, dann mit Aceton/Wasser 8 : 2 und schließlich mit Aceton/Wasser 7 : 3 eluiert. Die Fraktionen werden dünnschichtchromatographisch auf Kieselgelplatten geprüft (Fließmittel: Methanol/Chloroform/wäßriger Ammoniak 3 : 2 : 2).
Die Fraktionen, die die gesuchte Verbindung enthalten, werden zusammengefaßt und eingeengt.
Ausbeute: 2,5 g. Rf-Wert auf DC-Fertigplatten (Merck, Darmstadt, Kieselgel 60 F 254) mit Methanol/Chloroform/wäßriger Ammoniak 3 : 2 : 2 als Fließmittel: 0,525. Rf-Wert für 1-Desoxynojirimycin: 0,53.

Beispiel 11

Analog Beispiel 10 wurde erhalten:

1,8[N,N'-Bis-(1',5'-didesoxy-1',5'-imino-D-glucit)-yl]-n-octan

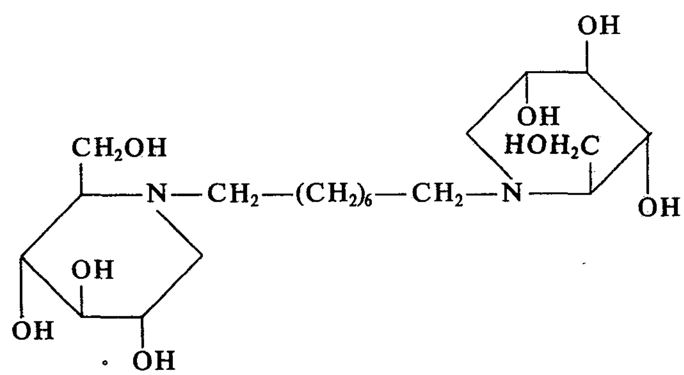

Rf-Wert: 0,63. Rf-Wert für 1-Desoxynojirimycin: 0,53.

Beispiel 12

3-N-(1',5'-didesoxy-1',5'-imino-D-glucityl)-propionsäure-1-α-(1",5"-didesoxy-1",5"-imino-D-glucityl)-methylamid

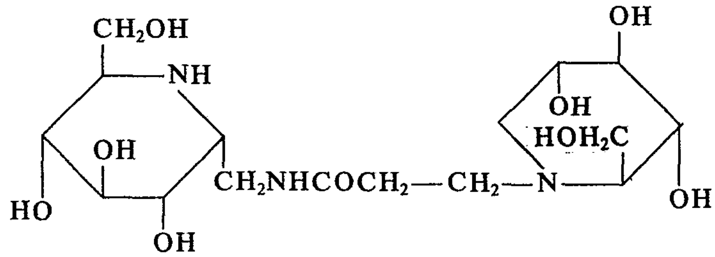

Zu 0,59 g 1-Desoxynojirimycinyl-β-propionsäure in 10 ml Pyridin und 7 ml $H_2O$ gibt man bei 20°C 0,52 g Dicyclohexylcarbodiimid. Man trägt 0,48 g 1-Aminomethyl-1-desoxynojirimycin in 3 ml $H_2O$ ein und rührt 12 Stunden bei Raumtemperatur. Anschließend erwärmt man auf 50°C und läßt weitere 20 Stunden rühren. Es wird eingeengt und der Rückstand in 25 ml $H_2O$ aufgenommen. Man saugt vom Unlöslichen ab und gibt die Lösung auf eine 25 cm lange und 3 cm weite Säule, die als Füllmittel Amberlite JRA 400 ($OH^{\ominus}$-Form) enthält. Es wird mit ca. 250 ml $H_2O$ eluiert, das wäßrige Eluat wird eingeengt.

Der Rückstand wird auf eine Säule aufgetragen, die als stationäre Phase Zellulose und als mobile Phase Aceton enthält. Es wird mit Aceton/$H_2O$ 9 : 1, dann mit Aceton/$H_2O$ 8 : 2 und schließlich mit Aceton/$H_2O$ 7 : 3 eluiert. Die einzelnen Fraktionen werden dünnschichtchromatographisch überprüft. Die Fraktionen mit dem gewünschten Produkt werden zusammengefaßt und eingeengt.

Ausbeute: 0,6 g. Rf-Wert: 0,25 (DC-Fertigplatten, Merck, Kieselgel 60 F 254, Fließmittel: Methanol/Chloroform/25%iger Ammoniak 3 : 2 : 2), Rf-Wert von 1-Aminomethyl-1-desoxynojirimycin = 0,28.

Beispiel 13

3-(N-1",5"-didesoxy-1",5"-imino-D-glucityl)-propionsäure-3'-(N'-1‴,5‴-didesoxy-1‴,5‴-imino-D-glucityl)-n-propylamid

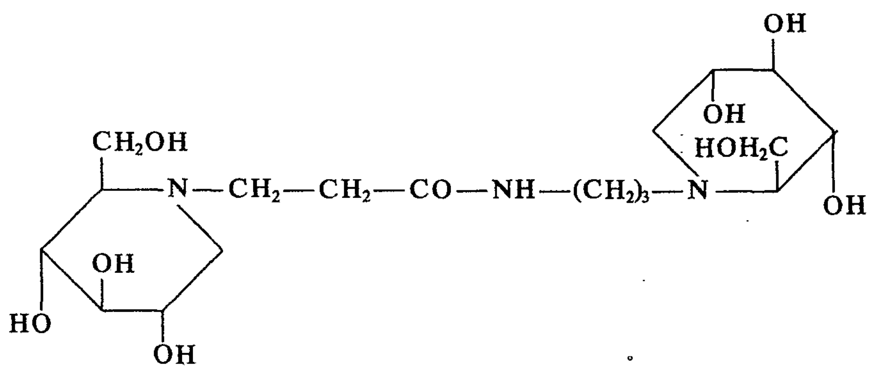

Zu 3 g 1-Desoxynojirimycinyl-β-propionsäure in 20 ml $H_2O$ und 20 ml Pyridin gibt man bei 20°C unter Rühren 0,1 g 4-Dimethylaminopyridin und 3,3 g γ-Amino-N-n-propyl-1-Desoxynojirimycin gelöst in 10 ml $H_2O$ und 10 ml Pyridin. Man trägt 2,9 g Dicyclohexylcarbodiimid ein und läßt 15 Stunden bei 20°C rühren.

Die Lösung wird eingeengt und der Rückstand auf eine mit Kieselgel gefüllte Säule aufgetragen.

Es wird mit Essigester/Methanol/Wasser/wäßriger Ammoniak 100 : 60 : 5 : 1 eluiert.

Die Fraktionen werden dünnschichtchromatographisch auf Kieselgelplatten geprüft (Fließmittel: Essigester/Methanol/Wasser/wäßriger Ammoniak 100 : 60 : 25 : 1).

Die Fraktionen, die die gesuchte Verbindung enthalten, werden zusammengefaßt und eingeengt.

Ausbeute: 1,4 g. Drehwert: $[\alpha]_{589} = -0{,}086$. Rf-Wert des Produkts/Rf-Wert von 1-Desoxynojirimycin-yl-β-propionsäure = 1 : 0,26 (DC-Fertigplatten Merck, Kieselgel 60 F 254, Fließmittel: Essigester/Methanol/Wasser/25%iger Ammoniak 100/60/25/1).

Analog Herstellungsbeispiel 4 wurden hergestellt:

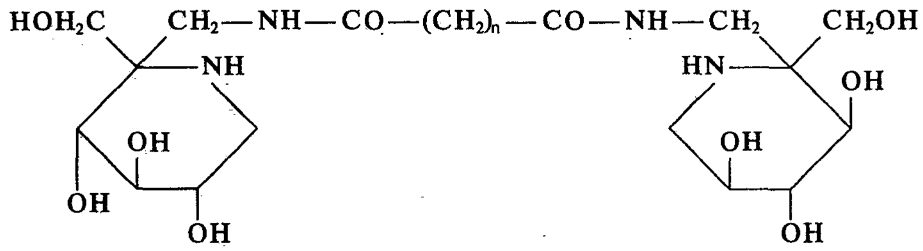

| Beispiel Nr. | n | Rf-Wert in Methanol/Chloroform/25%igen Ammoniak 3 : 2 : 2 (Rf-Wert = 0,34 für 2-Aminomethyl-2-hydroxymethyl-3,4,5-trihydroxypiperidin × 2 HCl) |
|---|---|---|
| 14 | 2 | 0,2 |
| 15 | 3 | 0,23 |
| 16 | 4 | 0,3 |
| 17 | 6 | 0,41 |
| 18 | 7 | 0,47 |

Beispiel 19

*α,α'*-[N,N'-Bis-(1,5-didesoxy-1,5-imino-D-glucit)-yl]-p-xylol

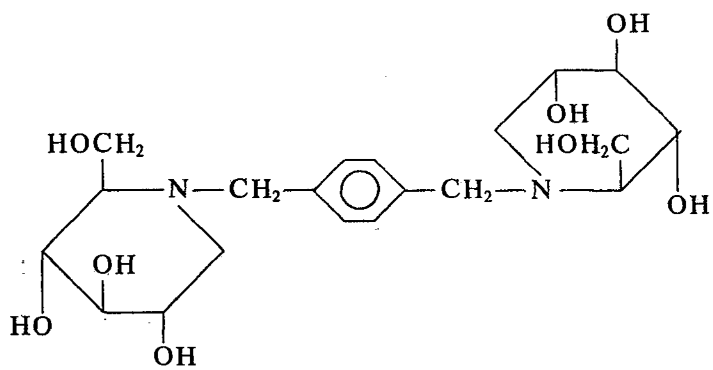

Die Verbindung wurde in Analogie zu Beispiel 6 aus *α,α'*-Dibromxylol und 1-Desoxynojirimycin erhalten.

Rf-Wert: 0,53. Rf-Wert für 1-Desoxynojirimycin: 0,51 (DC-Fertigplatten der Firma Merck, Darmstadt, Kieselgel 60 F 254; Fließmittel: Methanol/Chloroform/25%iger Ammoniak 3 : 2 : 2). F. P.: 280–281°C.

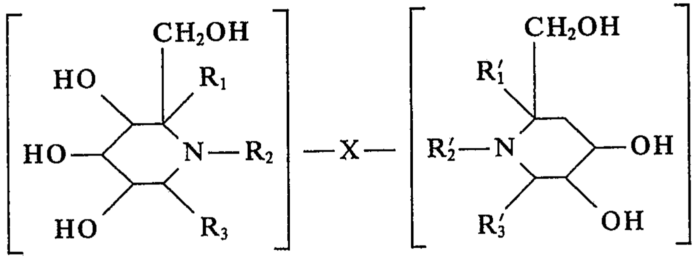

| $R_1$ | $R_2$ | $R_3$ | X | $R_1'$ | $R_2'$ | $R_3'$ |
|---|---|---|---|---|---|---|
| H | – | H | $-CH_2-CH=CH-(CH_2)_3-CH=CH-CH_2-$ | H | – | H |
| H | – | H | $-CH_2-CH=CH-(CH_2)_4-CH=CH-CH_2-$ | H | – | H |
| H | – | H | $-CH_2-CH=CH-(CH_2)_6-CH=CH-CH_2-$ | H | – | H |
| H | – | H | $-CH_2-CH=CH-(CH_2)_8-CH=CH-CH_2-$ | H | – | H |
| H | – | H | $-CH_2-CH=CH-CH_2-O-(CH_2)_2-O-CH_2-CH=CH-CH_2-$ | H | – | H |
| H | – | H | $-CH_2-CH=CH-CH_2-O-(CH_2)_3-O-CH_2-CH=CH-CH_2-$ | H | – | H |
| H | – | H | $-CH_2-CH=CH-CH_2-O-(CH_2)_4-O-CH_2-CH=CH-CH_2-$ | H | – | H |
| H | – | H | $-CH_2-CH=CH-CH_2-O-(CH_2)_5-O-CH_2-CH=CH-CH_2-$ | H | – | H |
| H | – | H | $-CH_2-CH=CH-CH_2-O-CH_2-CH=CH-CH_2-O-CH_2-CH=CH-CH_2-$ | H | – | H |
| H | – | H | $-CH_2-CH=CH-CH_2-O-CH_2-$ 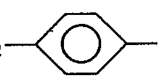 $-CH_2O-CH_2-CH=CH-CH_2-$ | H | – | H |
| H | – | H | $-CH_2-CH=CH-CO-NH-CH_2CH_2-NHCO-CH=CH-CH_2-$ | H | – | H |
| H | – | H | $-CH_2-CH=CHCONH-(CH_2)_4-NHCO-CH=CH-CH_2-$ | H | – | H |
| H | – | H | $-CH_2-CH=CHCO-NH-(CH_2)_6-NHCOCH=CH-CH_2-$ | H | – | H |
| H | – | H | $-CH_2-CH=CH-CH_2-NH-(CH_2)_4-NH-CH_2-CH=CH-CH_2-$ | H | – | H |

Fortsetzung

| $R_1$ | $R_2$ | $R_3$ | X | $R_1'$ | $R_2'$ | $R_3'$ |
|---|---|---|---|---|---|---|
| H | – | H | $-CH_2-CH=CH-CH_2-NHCONH-(CH_2)_6-NHCONH-CH_2-CH=CH-CH_2-$ | H | – | H |
| H | – | H | $-CH_2-CH=CH-\underset{\underset{O}{\Vert}}{C}-O-(CH_2)_4-O-\underset{\underset{O}{\Vert}}{C}-CH=CH-CH_2-$ | H | – | H |
| H | – | H | $-CH_2-CH=CH-CO-NH-CH_2-$ | H | H | – |
| H | – | H | $-CH_2-CH=CH-CO-NH-CH_2-$ | – | H | H |
| H | – | H | $-CH_2-CH=CH-CO-NH-(CH_2)_2-NH-CO-NH-CH_2-$ | H | H | – |
| H | – | H | $-CH_2-CH=CH-CH_2-NH-CO-CH_2-CH_2-CO-NH-CH_2-$ | H | H | – |
| H | – | H | $-CH_2-CH=CH-CH_2-NH-CO-(CH_2)_3-CO-NH-CH_2-$ | – | H | H |
| H | – | H | $-CH_2-CH=CH-CO-NH-CH_2-CH_2-CH_2-$ | H | – | H |
| H | – | H | $-CH_2-CH=CH-CH_2-CO-NH-CH_2-$ | H | H | – |
| H | – | H | $-CH_2-CH=CH-CH_2-CO-NH-CH_2-$ | – | H | H |
| H | – | H | $-CH_2-CH=CH-CH_2-CO-NH-(CH_2)_4-NH-CO-NH-CH_2-$ | H | H | – |
| H | – | H | $-CH_2-CH=CH-CH_2-CO-NH-(CH_2)_6-NH-CO-NH-CH_2-$ | – | H | H |
| H | – | H | $-CH_2-CH=CH-CH_2-NH-CO-NH-CH_2-$ | H | H | – |
| H | – | H | $-CH_2-CH=CH-CH_2-NH-CO-NH-CH_2-$ | – | H | H |
| H | – | H | $-CH_2-CH=CH-CH_2-NH-SO_2-NH-CH_2-$ | H | H | – |
| H | – | H | $-CH_2-CH=CH-CH_2-NH-SO_2-NH-CH_2-$ | – | H | H |
| H | – | H | $-CH_2-CH_2-CH_2-NH-CO-NH-CH_2-$ | H | H | – |

Fortsetzung

| $R_1$ | $R_2$ | $R_3$ | X | $R_1'$ | $R_2'$ | $R_3'$ |
|---|---|---|---|---|---|---|
| H | – | H | $-CH_2-CH_2-CH_2-NH-CO-NH-CH_2-$ | – | H | H |
| H | – | H | $-CH_2-CH_2-CH_2-NH-SO_2-NH-CH_2-$ | H | H | – |
| H | – | H | $-CH_2-CH_2-CH_2-NH-SO_2-NH-CH_2-$ | – | H | H |
| H | – | H | $-CH_2-CH_2-CH_2-NH-CO-CH_2-CH_2-CO-NH-CH_2-$ | H | H | – |
| H | – | H | $-CH_2-CH_2-CH_2-NH-CO-CH_2-CH_2-CO-NH-CH_2-$ | – | H | H |
| H | – | H | $-CH_2-CH_2-CH_2-NH-CO-NH-(CH_2)_2-NH-CO-NH-CH_2-$ | H | H | – |
| H | – | H | $-CH_2CH_2CH_2-NHCONH-CH_2CH_2CH_2-$ | H | – | H |
| H | – | H | $-CH_2-CH_2-CH_2-NH-CO-NH-(CH_2)_3-NH-CO-NH-CH_2-$ | – | H | H |
| H | H | – | $-CH_2-NH-CO-NH-SO_2-NH-CH_2-$ | H | H | – |
| H | H | – | $-CH_2-NH-CO-NH-SO_2-NH-CH_2-$ | – | H | H |
| H | H | – | $-CH_2-NH-CO-NH-CO-NH-CH_2-$ | H | H | – |
| H | H | – | $-CH_2-NH-CO-NH-CO-NH-CH_2-$ | – | H | H |
| H | – | H | $-CH_2-(CH=CH)_2-CO-NH-CH_2-$ | H | H | – |
| H | – | H | $-CH_2-(CH=CH)_2-CO-NH-CH_2-$ | – | H | H |
| H | – | H | $-CH_2-(CH=CH)_2-CO-NH-CH_2-CH_2-CH_2-$ | H | – | H |
| H | – | H | $-CH_2-(CH=CH)_2-CO-NH-(CH_2)_2-NH-CO-NH-CH_2-$ | H | H | – |
| H | – | H | $-CH_2-(CH=CH)_2-CO-NH-(CH_2)_6-NH-CO-NH-CH_2-$ | – | H | H |
| H | – | $CH_3$ | $-CH_2-NH-CO-NH-(CH_2)_6-NH-CO-NH-CH_2-$ | H | H | – |

Fortsetzung

| $R_1$ | $R_2$ | $R_3$ | X | $R'_1$ | $R'_2$ | $R'_3$ |
|---|---|---|---|---|---|---|
| – | $CH_3$ | H | $-CH_2-NH-CO-NH-(CH_2)_6-NH-CO-NH-CH_2-$ | – | H | H |
| H | $CH_3$ | – | $-CH_2-NH-CO-(CH_2)_8-CO-NH-CH_2-$ | H | H | – |
| – | $CH_3$ | H | $-CH_2-NH-CO-(CH_2)_8-CO-NH-CH_2-$ | – | H | H |
| H | H | – | 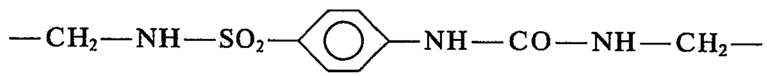 | – | H | H |
| H | $CH_3$ | – | 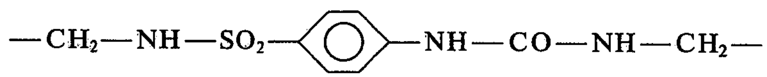 | – | H | H |
| H | H | – | 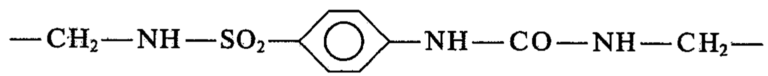 | – | $CH_3$ | H |
| H | H | – | 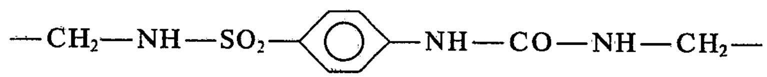 | H | H | – |
| – | H | H | 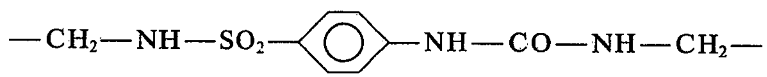 | – | H | H |
| – | H | H | 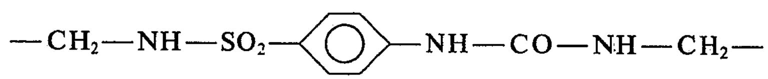 | H | H | – |
| H | H | – | 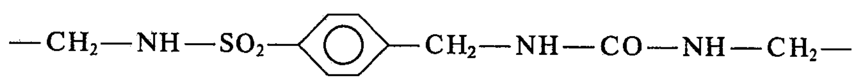 | – | H | H |
| – | H | H | 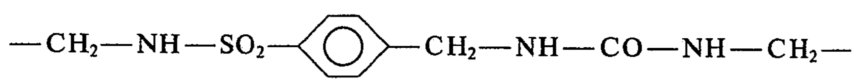 | H | H | – |
| H | $CH_3$ | – | 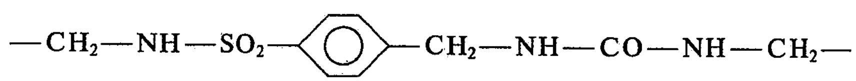 | – | H | H |
| H | H | – | 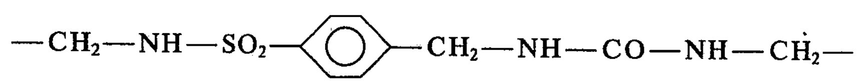 | – | $CH_3$ | H |

Fortsetzung

| R₁ | R₂ | R₃ | X | R′₁ | R′₂ | R′₃ |
|---|---|---|---|---|---|---|
| H | – | H | $-CH_2-(CH_2)_2-CH_2-$ | H | – | H |
| H | – | H | $-CH_2-CH_2-$ | H | – | H |
| H | – | H | $-CH_2-(CH_2)_5-CH_2-$ | H | – | H |
| H | – | H | $-CH_2-CH_2-CH_2-$ | H | – | H |
| H | – | H | $-CH_2-(CH_2)_7-CH_2-$ | H | – | H |
| H | – | H | $-CH_2-(CH_2)_8-CH_2-$ | H | – | H |
| H | – | H | 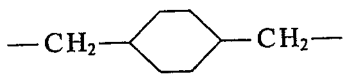 | H | – | H |
| H | – | H | $-(CH_2)_2-CO-O-CO-(CH_2)_2-$ | H | – | H |
| H | – | H | $-CH(CH_3)-CH_2-CH_2-CO-O-CH_2-(CH_2)_4-CH_2-$ | H | – | H |
| H | – | H | 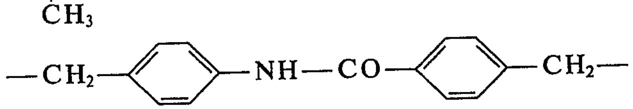 | H | – | H |
| H | – | H | $-CH_2-CH(OH)-CH_2-O-CH_2-CH(OH)-CH_2-$ | H | – | H |
| H | – | H | $-CH_2-(CH_2)_4-O-CO-NH-CH_2-$ | – | H | H |
| H | – | H | $-CH_2-(CH_2)_4-O-CO-NH-CH_2-$ | – | $CH_3$ | H |
| H | – | H | $-CH_2-(CH_2)_4-O-CO-NH-CH_2-$ | H | H | – |
| H | – | H | $-CH_2-(CH_2)_9-O-CO-NH-CH_2-$ | – | H | H |
| H | – | H | $-CH_2-(CH_2)_9-O-CO-NH-CH_2-$ | H | H | – |
| H | – | H | 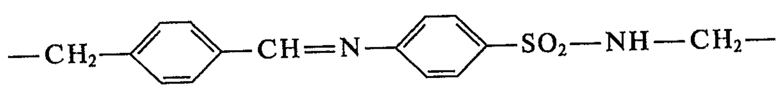 | – | H | H |

Fortsetzung

| $R_1$ | $R_2$ | $R_3$ | X | $R_1'$ | $R_2'$ | $R_3'$ |
|---|---|---|---|---|---|---|
| H | – | H | 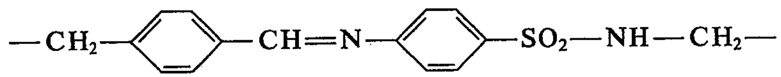 | H | H | – |
| H | – | H | 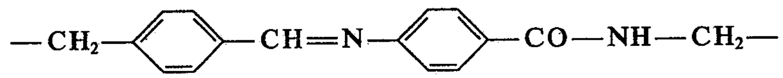 | H | H | – |
| H | – | H | 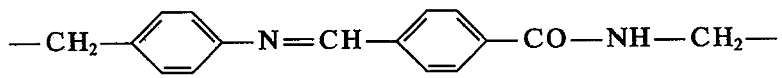 | – | H | H |
| H | – | H | $-CH(CH_3)-(CH_2)_2-CO-NH-(CH_2)_3-$ | H | – | H |
| H | – | H | $-(CH_2)_6-NH-CO-(CH_2)_2-$ | H | – | H |
| H | – | H | $-(CH_2)_5-CO-NH-CH_2-$ | – | H | H |
| H | – | H | $-(CH_2)_5-CO-NH-CH_2-$ | H | H | – |
| H | – | H | $-(CH_2)_3-NH-CO-NH-(CH_2)_6-NH-CO-NH-(CH_2)_3-$ | H | – | H |
| H | – | H | $-(CH_2)_6-NH-CO-NH-(CH_2)_6-NH-CO-NH-(CH_2)_6-$ | H | – | H |
| H | – | H | $-(CH_2)_6-NH-CS-NH-(CH_2)_6-NH-CS-NH-(CH_2)_6-$ | H | – | H |
| H | – | H | $-(CH_2)_2-CO-NH-(CH_2)_6-NH-CO-(CH_2)_2-$ | H | – | H |
| H | – | H | 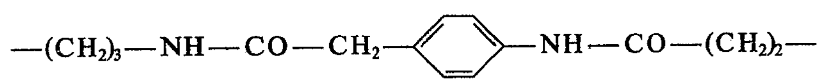 | H | – | H |
| H | – | H | 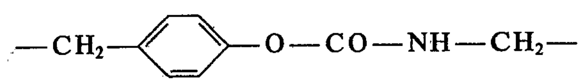 | – | H | H |
| H | – | H | 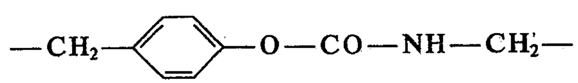 | H | H | – |

Fortsetzung

| $R_1$ | $R_2$ | $R_3$ | X | $R_1'$ | $R_2'$ | $R_3'$ |
|---|---|---|---|---|---|---|
| H | – | H | 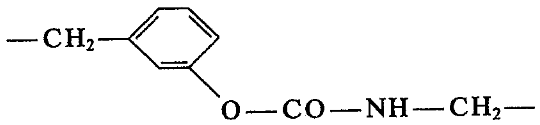 | – | H | H |
| H | – | H | 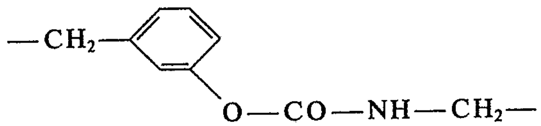 | H | H | – |
| H | – | H | 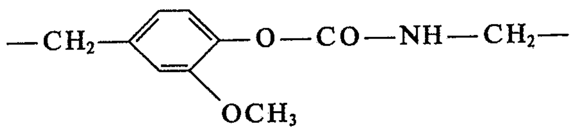 | – | H | H |
| H | – | H | 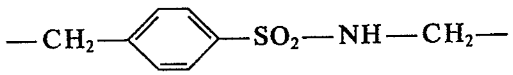 | – | H | H |
| H | – | H | 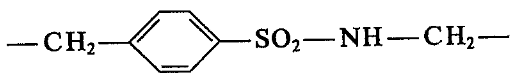 | H | H | – |
| H | – | H | 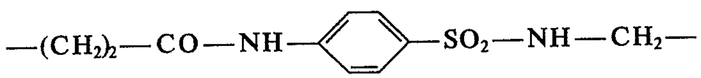 | H | H | – |
| H | – | H | 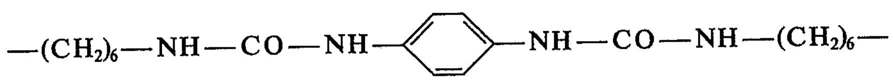 | H | – | H |
| H | – | H | 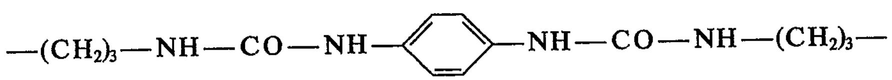 | H | – | H |
| H | – | H | 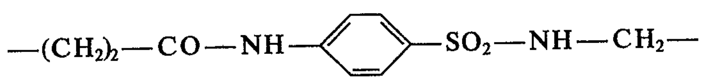 | – | H | H |
| H | – | H | 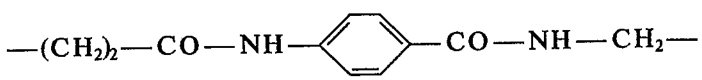 | – | H | H |

Fortsetzung

| $R_1$ | $R_2$ | $R_3$ | X | $R'_1$ | $R'_2$ | $R'_3$ |
|---|---|---|---|---|---|---|
| H | – | H | 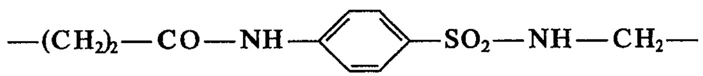 | H | H | – |
| H | – | H | 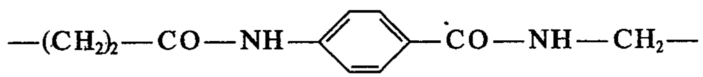 | H | H | – |
| H | – | H | 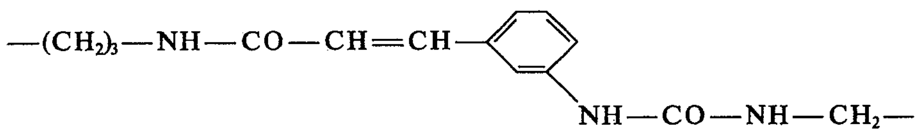 | – | H | H |
| H | – | H | $-CH_2-NH-CO-O-(CH_2)_4-O-CO-NH-CH_2-$ | – | H | H |
| H | – | H | $-CH_2-NH-CO-O-(CH_2)_5-O-CO-NH-CH_2-$ | – | H | H |
| – | H | H | 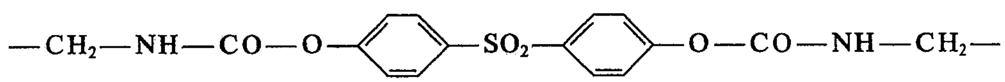 | – | H | H |
| – | H | H | 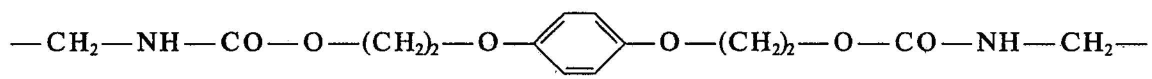 | – | H | H |
| – | H | H | 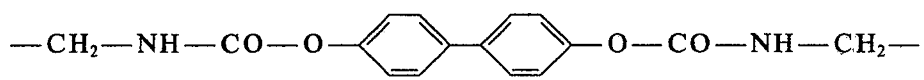 | – | H | H |
| H | H | – | 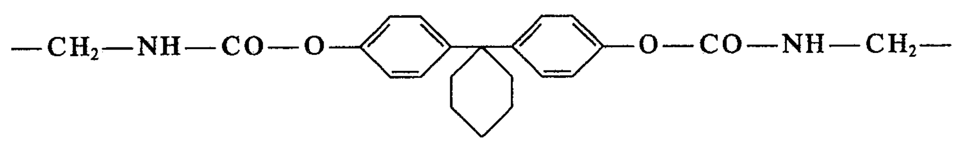 | H | H | – |
| – | H | H | 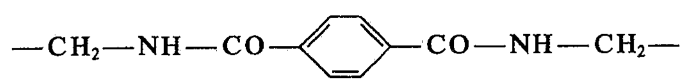 | – | H | H |
| H | H | – | 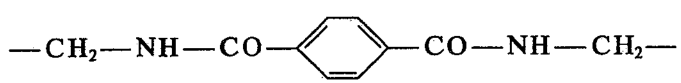 | H | H | – |

Fortsetzung

| $R_1$ | $R_2$ | $R_3$ | X | $R'_1$ | $R'_2$ | $R'_3$ |
|---|---|---|---|---|---|---|
| – | H | H | 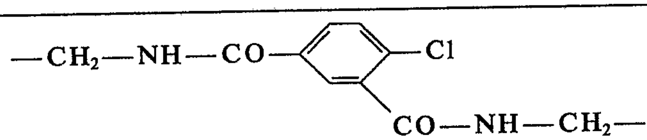 | – | H | H |
| – | H | H | 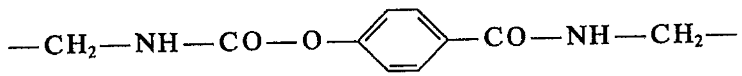 | – | H | H |
| – | H | H | 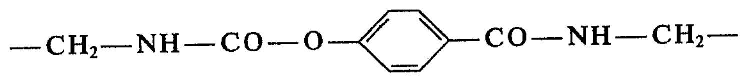 | H | H | – |
| – | H | H | 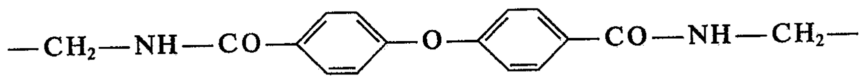 | – | H | H |
| H | H | – | 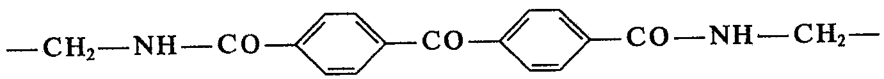 | H | H | – |
| – | H | H | 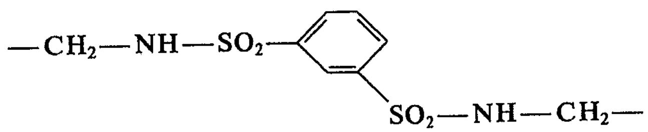 | – | H | H |
| H | H | – | 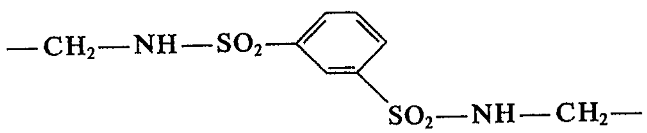 | H | H | – |
| – | H | H | 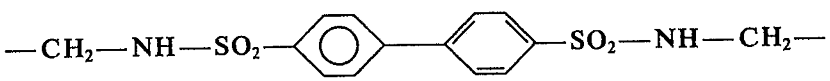 | – | H | H |
| H | H | – | 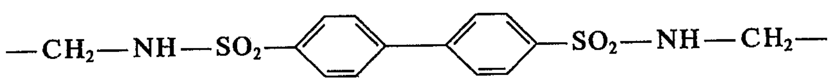 | H | H | – |
| H | H | – | $—CH_2—NH—CO—O—(CH_2)_5—O—CO—NH—CH_2—$ | H | H | – |
| H | H | – | $—CH_2—NH—CO—O—(CH_2)_4—O—CO—NH—CH_2—$ | H | H | – |

Fortsetzung

| $R_1$ | $R_2$ | $R_3$ | X | $R_1'$ | $R_2'$ | $R_3'$ |
|---|---|---|---|---|---|---|
| – | H | H | $-CH_2-NH-CO-O-(CH_2)_2-O-CO-NH-CH_2-$ | – | H | H |
| – | H | H | $-CH_2-NH-CO-O-(CH_2)_2-O-(CH_2)_2-O-(CH_2)_2-O-CO-NH-CH_2-$ | – | H | H |
| H | H | – | $-CH_2-NH-CO-O-(CH_2)_2-O-(CH_2)_2-O-CO-NH-CH_2-$ | H | H | – |
| – | H | H | $-CH_2-NH-CO-O-CH_2-CH{=}CH-CH_2-O-CO-NH-CH_2-$ | – | H | H |
| – | H | H | $-CH_2-NH-CO-O-CH_2-CH_2-CH_2-O-CO-NH-CH_2-$ | – | H | H |
| – | H | H | $-CH_2-NH-CO-CH{=}CH-CO-NH-CH_2-$ | – | H | H |
| – | H | H | $-CH_2-NH-CS-NH-(CH_2)_5-NH-CS-NH-CH_2-$ | – | H | H |
| – | H | H | 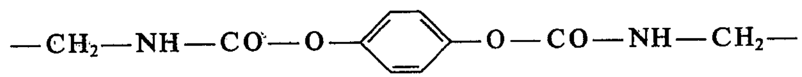 | – | H | H |
| H | H | – | 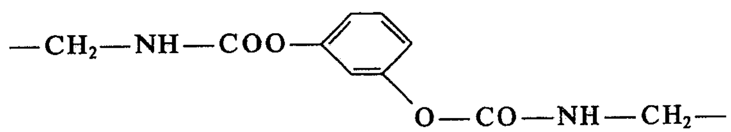 | H | H | – |
| – | H | H | 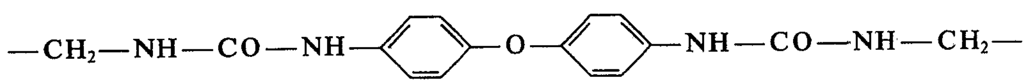 | – | H | H |
| H | H | – | 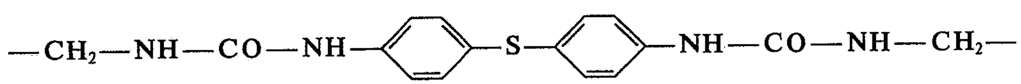 | H | H | – |
| – | H | H | 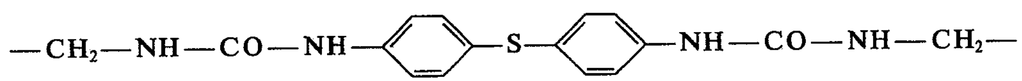 | – | H | H |
| – | H | H | 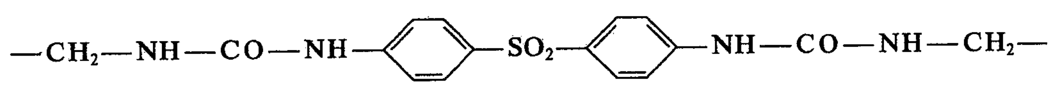 | – | H | H |
| – | H | H | 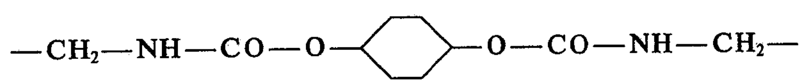 | – | H | H |

Fortsetzung

| $R_1$ | $R_2$ | $R_3$ | X | $R'_1$ | $R'_2$ | $R'_3$ |
|---|---|---|---|---|---|---|
| – | H | H | 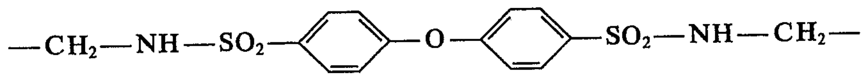 | — | H | H |
| – | H | H | 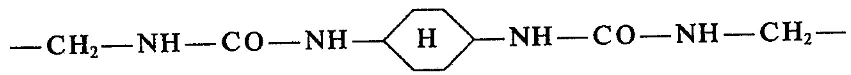 | — | H | H |
| H | H | – | 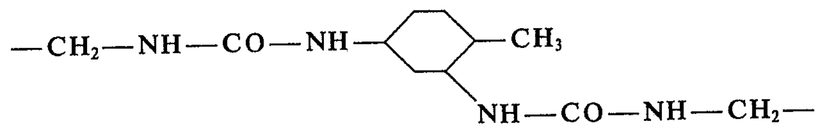 | H | H | – |
| – | H | H | 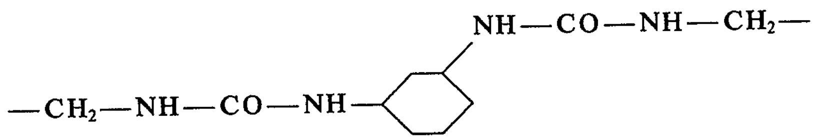 | — | H | H |
| – | H | H | 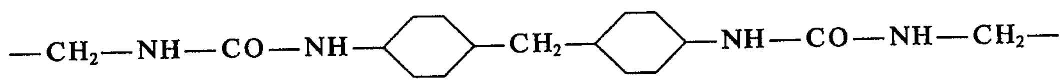 | — | H | H |
| H | H | – | 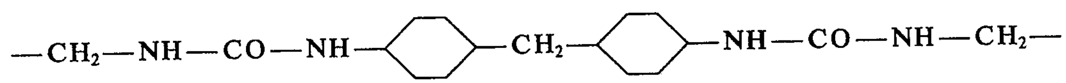 | H | H | – |
| – | H | H | 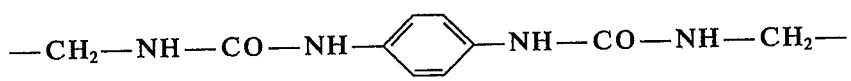 | — | H | H |
| – | H | H | 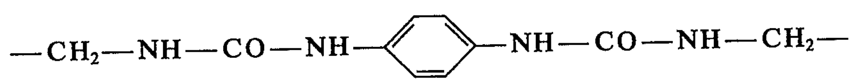 | H | H | – |
| H | H | – | 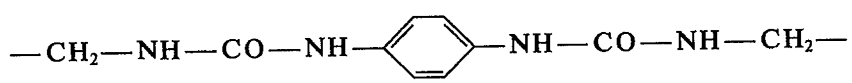 | H | H | – |
| – | H | H | 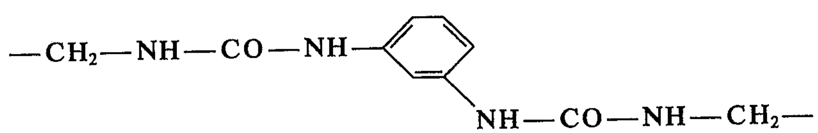 | – | H | H |

Fortsetzung

| $R_1$ | $R_2$ | $R_3$ | X | $R_1'$ | $R_2'$ | $R_3'$ |
|---|---|---|---|---|---|---|
| – | H | H | 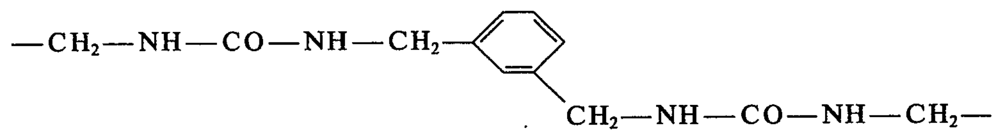 | – | H | H |
| H | H | – | 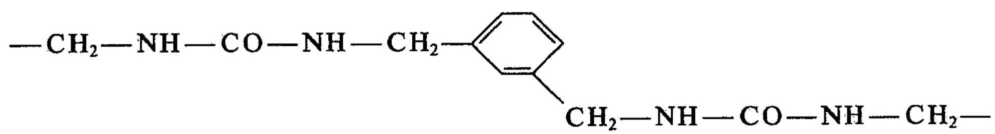 | H | H | – |
| – | H | H | 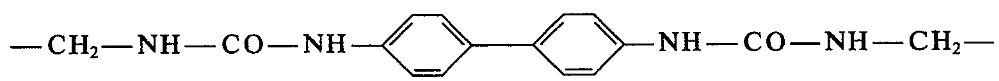 | – | H | H |
| – | H | H | 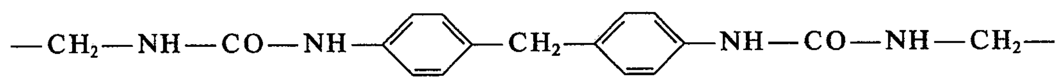 | – | H | H |
| – | H | H | 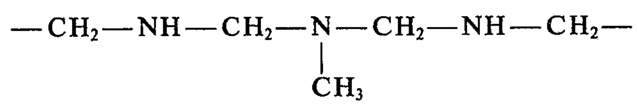 | – | H | H |
| H | H | – | 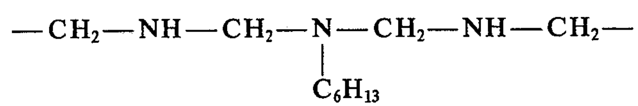 | H | H | – |

Analog Beispiel 9, aber mit Bisisocyanaten, wurden erhalten:

Beispiel 20

$N^1,N^4$-Bis-[5-(1,5-imino-1,5-didesoxy-D-glucityl)-methyl]-4,4'-oxy-bisphenylharnstoff

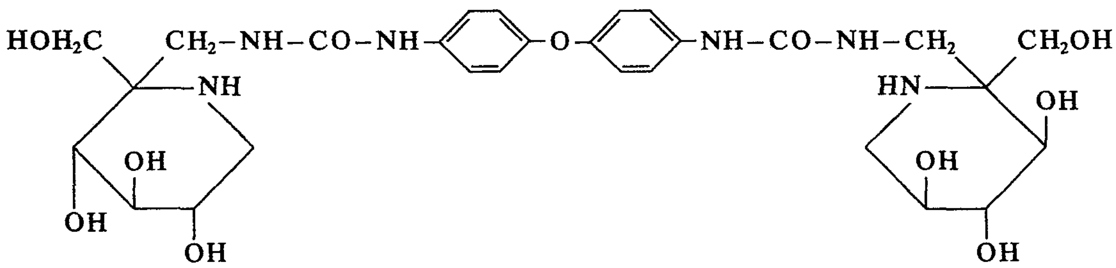

Rf-Wert: 0,51. Rf-Wert für 2-Aminomethyl-2-hydroxymethyl-3,4,5-trihydroxy-piperidin-dihydrochlorid = 0,36. Fließmittel: Methanol/Chloroform/25%iger Ammoniak im Volumenverhältnis 3 : 2 : 2.

Beispiel 21

$N^1,N^4$-Bis-[5-(1,5-imino-1,5-didesoxy-D-glucityl)-methyl]-phenylen-1,4-bisharnstoff

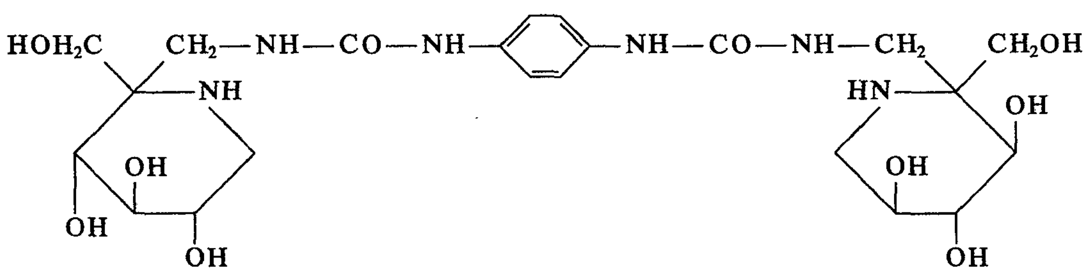

Rf-Wert: 0,3. Vergleich wie Beispiel 20.

Beispiel 22

$N^1,N^4$-Bis-[5-(1,5-imino-1,5-didesoxy-D-glucityl)-methyl]-(4,6-dimethyl-phenylen-1,3-methylen-bisharnstoff)

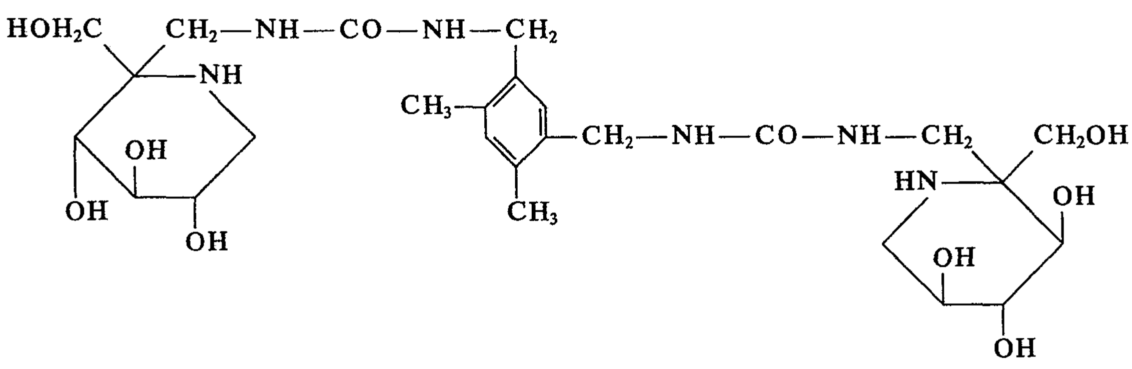

Rf-Wert = 0,48. Vergleich wie Beispiel 20.

Beispiel 23

$N^1$,$N^4$-Bis-[5-(1,5-imino-1,5-didesoxy-D-glucityl)-diphenylmethan]-4,4'-bisharnstoff

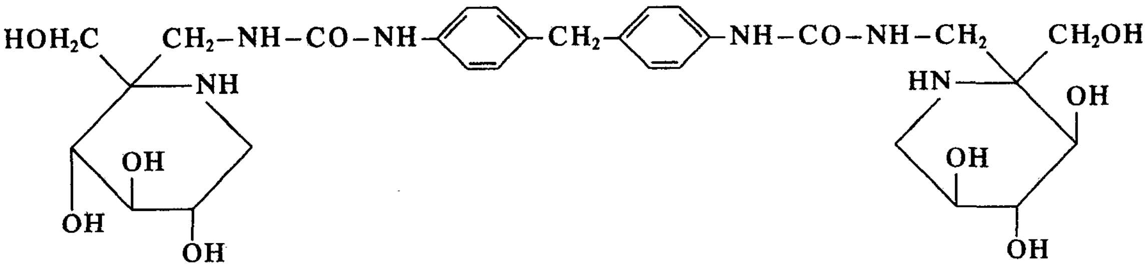

Rf-Wert: 0,55. Vergleich wie Beispiel 20.

Beispiel 24

$N^1$,$N^4$-Bis-[1-$\alpha$-(1,5-didesoxy-1,5-imino-D-glucityl)-methyl]-1,4-bisureido-cyclohexan

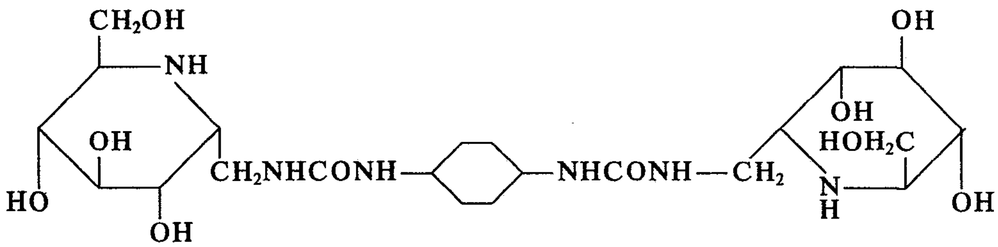

Von der Verbindung wurde ein $^{13}C$-Spektrum in $D_2O$ gemessen (Standard:

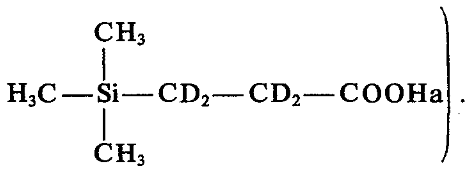

Die 10 verschiedenen C-Atome der Verbindung absorbieren bei (ppm bezogen auf Standard): 34 218, 38 503, 51 326, 57 040, 58 339, 64 670, 74 540, 74 865, 76 844 u. 162 587.

Beispiel 25

$N^1$,$N^4$-Bis-[1-$\alpha$-(1,5-didesoxy-1,5-imino-D-glucityl)-methyl]-4,4'-bisureido-diphenylmethan

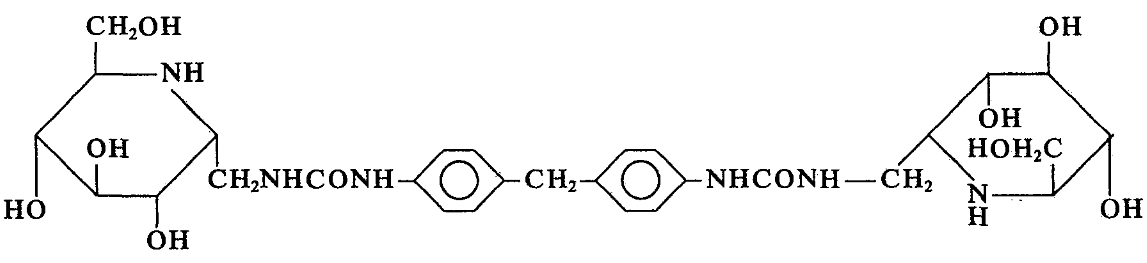

F. P.: 239°C.

Beispiel 26

N[1],N[4]-Bis-[1-*α*-(1,5-didesoxy-1,5-imino-D-glucityl)-methyl]-1,3-phenylen-bisharnstoff

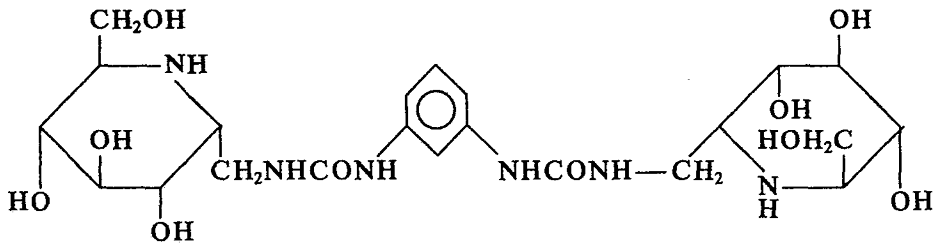

Von der Verbindung wurde ein $^{13}C$-Spektrum in $D_2O$ gemessen (Standard:

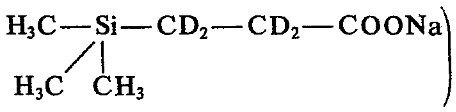

Die 12 verschiedenen C-Atome der Verbindung absorbieren bei (ppm bezogen auf Standard): 38 503, 57 073, 58 339, 64 670, 74 571, 74 896, 76 844, 155 478, 118 496, 132 360, 141 710 u. 160 571.

Beispiel 27

N[1],N[4]-Bis-[1-*α*-(1,5-didesoxy-1,5-imino-D-glucityl)-methyl]-*α*,*α*'-bisureido-m-xylol

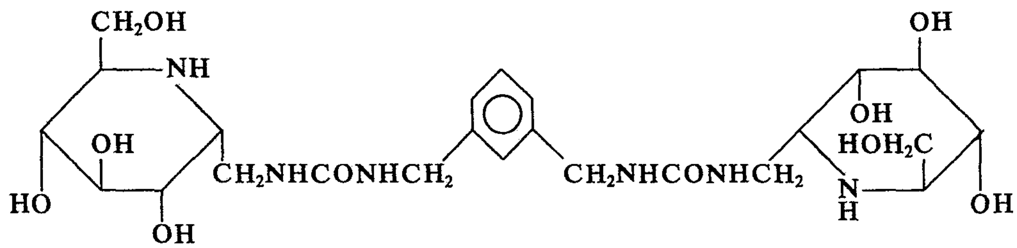

Rf-Wert: 0,28; 1-Aminomethyl-1-desoxynojirimycin: 0,23 (DC-Platten u. -Fließmittel wie in Beispiel 2).

Beispiel 28

N[1],N[4]-Bis-[1-*α*-(1,5-didesoxy-1,5-imino-D-glucityl)-methyl]-1,5-bisureido-naphthalin

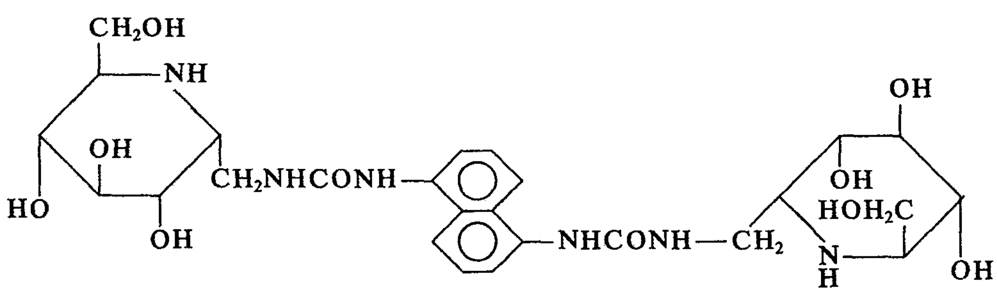

F. P.: 228°C.

Beispiel 29

$N^1$,$N^4$-Bis-[1-α-(1,5-didesoxy-1,5-imino-D-glucityl)-methyl]-1,4-phenylen-bisharnstoff

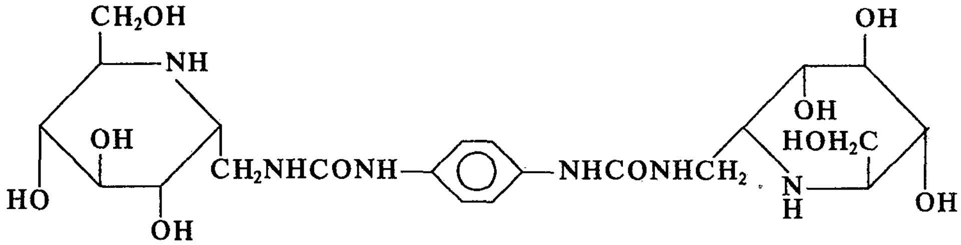

Rf-Wert: 0,21; 1-Aminomethyl-1-desoxynojirimycin: 0,23 (DC-Platten u. -Fließmittel wie in Beispiel 2).

Beispiel 30

Analog Beispiel 12 wurde aus 1-Desoxynojirimycinyl-β-propionsäure und 2-Aminomethyl-2-hydroxymethyl-3,4,5-trihydroxy-piperidin-dihydrochlorid

N-[(2-Hydroxymethyl-3,4,5-trihydroxy)-piperidin-2-methyl]-1-desoxynojirimycinyl-β-propionsäureamid

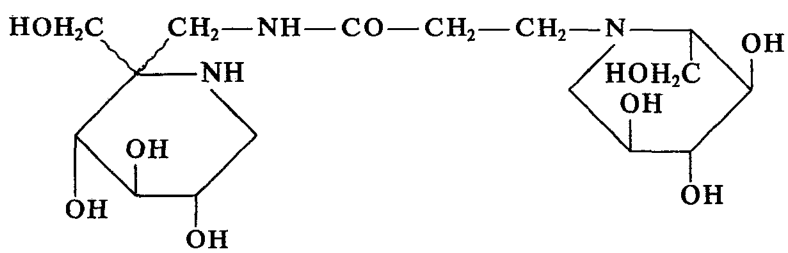

hergestellt.

Die wichtigsten Massenpeaks sind: m/e = 247 (M-162); m/e = 215; m/e = 162 (M-247).

Rf-Wert = 0,29. Rf-Wert von 2-Aminomethyl-2-hydroxymethyl-3,4,5-trihydroxypiperidin-dihydrochlorid = 0,36. Fließmittel: Methanol/Chloroform/25%iger Ammoniak im Volumenverhältnis 3 : 2 : 2.

Beispiel 31

N,N'-{3-[(1',5'-didesoxy-1',5'-imino-D-glucit-yl)-propionsäure]}-trimethylen-diamid

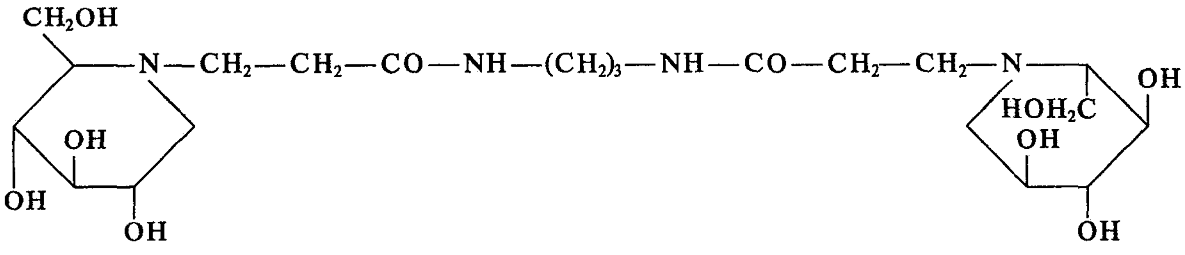

4,7 g 1-Desoxynojirimycinyl-β-propionsäure werden in 100 ml abs. Pyridin und 56 ml Wasser mit 0,86 ml 1,3-Diaminopropan und 6,24 g Dicyclohexylcarbodiimid 5 Tage bei 50°C gerührt. Es wird abgekühlt und abgesaugt. Das Filtrat wird eingeengt, in Wasser gelöst, abgesaugt und wieder eingeengt. Der Eindampfrückstand wird in wenig Wasser gelöst und über eine Zellulose-Säule mit wäßrigem Aceton gereinigt. Man erhält 0,5 g reines Produkt in Form eines Schaumes.

Rf-Wert: 0,45. Vergleich wie Beispiel 20.

Analog wurde hergestellt:

Beispiel 32

N,N'-[1-Desoxynojirimycinyl-(β-propionsäure)]-p-phenylenbisamid

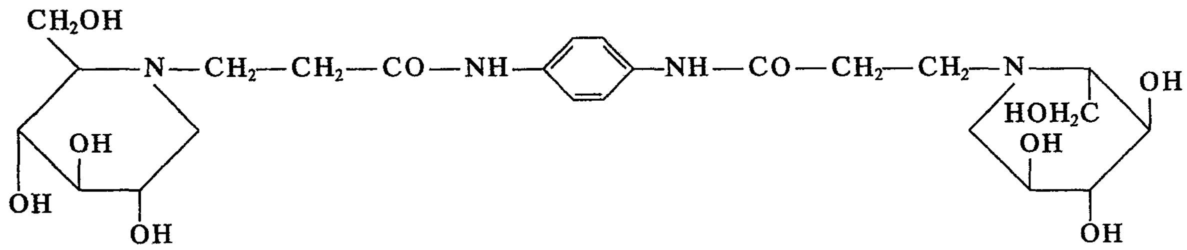

Rf-Wert = 0,53. Vergleich wie Beispiel 20.

Beispiel 33

N,N'-Bis-[1,2-(1,5-imino-1,5-didesoxy-D-glucit-yl)-methyl]-benzol-1,3-disulfonamid-dihydrochlorid

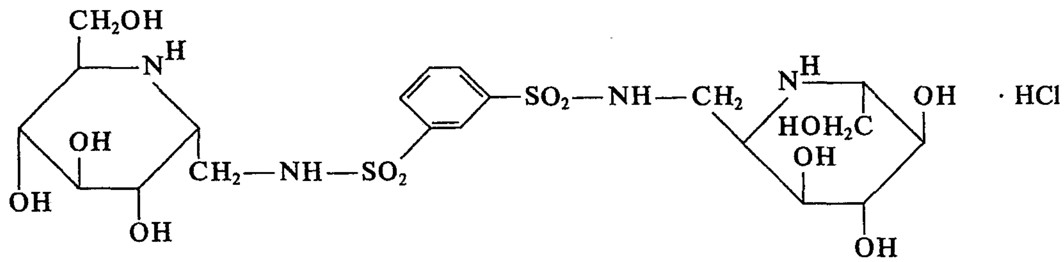

3 g 1-Aminomethyl-1-desoxynojirimycin werden in einer Mischung von 30 ml Wasser und 90 ml Aceton und nach Zugabe von 2,1 ml Triethylamin mit einer Lsg. von 2,04 g Benzol-1,3-disulfonsäurechlorid in 30 ml Aceton tropfenweise versetzt. Es wird 24 Stdn. gerührt, eingeengt, in ca. 1 Lit. Wasser gelöst und mit Anionenaustauscher Amberlite IRA 400 versetzt, bis die wäßrige Lösung chloridfrei ist. Es wird abgesaugt und der Austauscher gut mit Wasser gewaschen. Anschließend wird der Austauscher mit 5% Salzsäure verrührt und abgesaugt. Das Filtrat wird eingeengt; es wird in wenig Wasser gelöst und über eine Zellulosesäule mit wäßrigem Aceton gereinigt. Die sauberen Fraktionen werden eingeengt. Das Produkt kristallisiert bei mehrmaligem Einengen mit Isopropanol. Man erhält 2,1 g farblose Kristalle vom Schmelzpunkt ab 118°C unter Zersetzung.

Beispiel 34

N,N'-Bis-[β-(N-1-desoxynojirimycinyl)-(ethyl-sulfonylethyl)]-piperazin

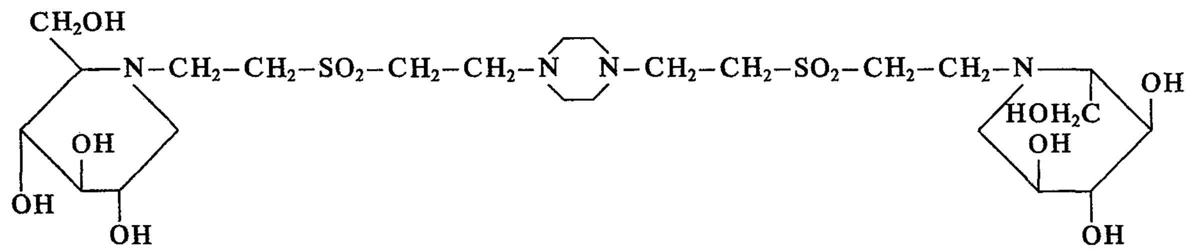

1,94 g Piperazin werden in 15 ml Wasser gelöst und tropfenweise mit einer Lösung von 12,6 g N-(Vinylsulfonyl-ethyl)-1-desoxynojirimycin (hergestellt aus 1-Desoxynojirimycin und Divinylsulfon, Schmp.: 156°C) in 50 ml Wasser versetzt. Es wird 18 Stdn. gerührt, eingeengt, in wenig Wasser gelöst und über eine Zellulosesäule mit wäßrigem Aceton gereinigt. Die sauberen Fraktionen werden gesammelt und eingeengt. Der Eindampfrückstand wird in warmem Methanol gelöst, filtriert und eingeengt, wobei Kristallisation eintritt. Es wird mit Methanol verrührt und abgesaugt. Man erhält 10,0 g farblose Kristalle vom Schmelzpunkt ab 175°C Z.

Beispiel 35

N,N'-[3,3'-(1,4-Phenylen)-bis-(2-propenyl)]-di-(1-desoxynojirimycin)

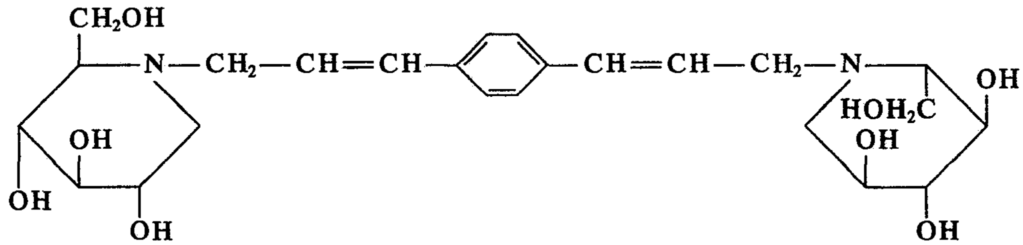

Zu 14,1 g 1-Desoxynojirimycin in 425 ml Methanol und 29 ml Eisessig wird eine Lösung von 9 g β,β'-p-Phenylen-di-acrolein in 270 ml Tetrahydrofuran gegeben. Es wird auf 0—5°C abgekühlt und auf einmal mit 11,6 g Natriumcyanoborhydrid versetzt. Es wird über Nacht bei 20°C gerührt und anschließend eingeengt. Der Eindampfrückstand wird in 400 ml Methanol/Wasser im Volumenverhältnis 8 : 1 gelöst und auf eine 30 cm lange und 6 cm weite Säule aufgetragen, die Kationenaustauscher Amberlite IR 120 (Serva) enthält. Es wird gut mit Methanol/Wasser im Volumenverhältnis 8 : 1 gewaschen und anschließend mit 2%igem Ammoniak eluiert. Die Fraktionen, die das gewünschte Produkt enthalten, werden gesammelt und eingeengt. Das erhaltene Rohprodukt wird danach über eine Zellulose-Säule wie oben beschrieben gereinigt. Das Produkt kristallisiert beim Einengen mit Methanol.

Man erhält 4,1 g farblose Kristalle vom Schmelzpunkt 258—260°C unter Zersetzung.

Beispiel 36

Analog Beispiel 6 wurde aus 1-Desoxynojirimycin und 1,4-Dichlorbutin-2 hergestellt:

1,4-[N,N'-Bis-(1,5-didesoxy-1',5'-imino-D-gluat)-yl]-butin-2

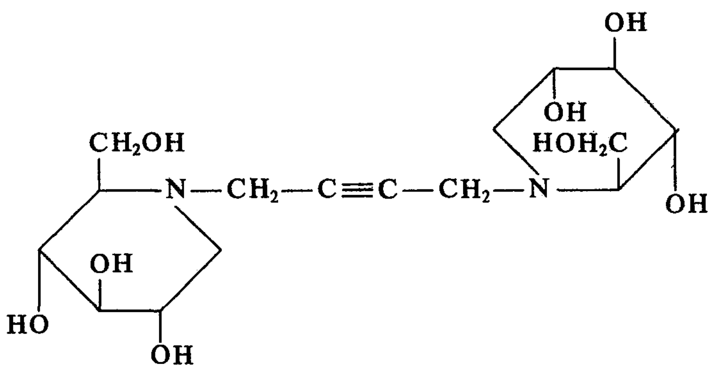

F. P.: 225°C (Zersetzung). Rf-Wert: 0,25; 1-Desoxynojirimycin: 0,42 (DC-Platten und -Fließmittel wie in Beispiel 2).

Die folgenden Verbindungen wurden analog zu Beispiel 4 hergestellt:

Beispiel 37

N,N'-Bis-[5-(1,5-imino-1,5-didesoxy-D-glucityl)-methyl]-fumarsäurediamid

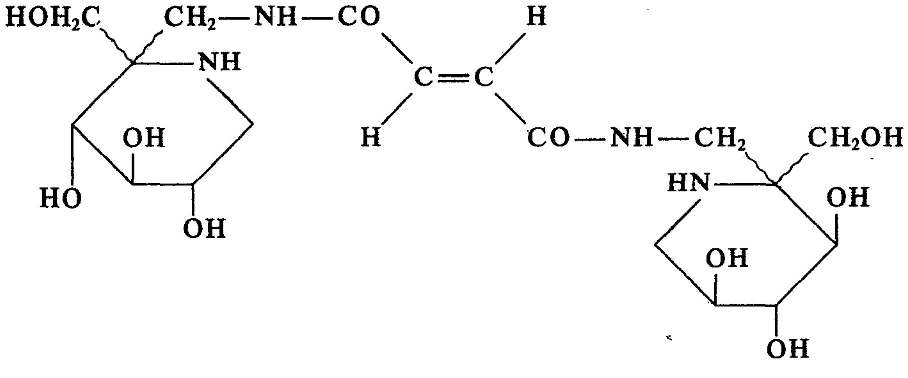

$R_f$-Wert: 0,19. $R_f$-Wert 2-Aminomethyl-3,4,5-trihydroxy-piperidin dihydrochlorid: 0,31 (Dünnschichtplatten und Laufmittel wie in Beispiel 2).

Beispiel 38

N,N'-Bis-[5-(1,5-imino-1,5-didesoxy-D-glucityl)-methyl]-3-hexendicarbonsäurediamid

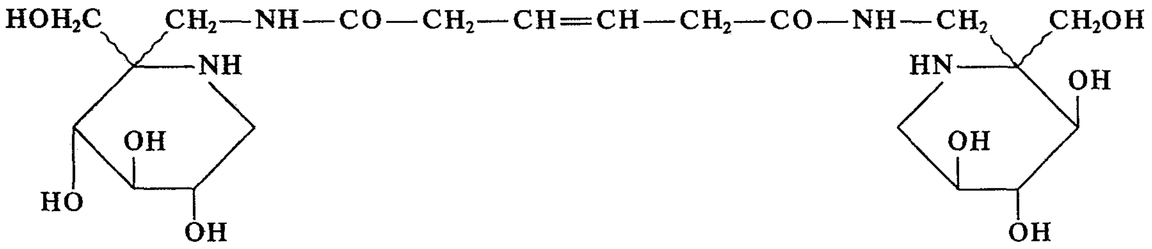

$R_f$-Wert: 0,23.
Die folgenden Verbindungen wurden analog Beispiel 9 hergestellt:

Beispiel 39

$N^1$,$N^4$-Bis-[5-(1,5-didesoxy-1,5-imino-D-glucityl)-methyl]-1,5-bisureido-naphthalin

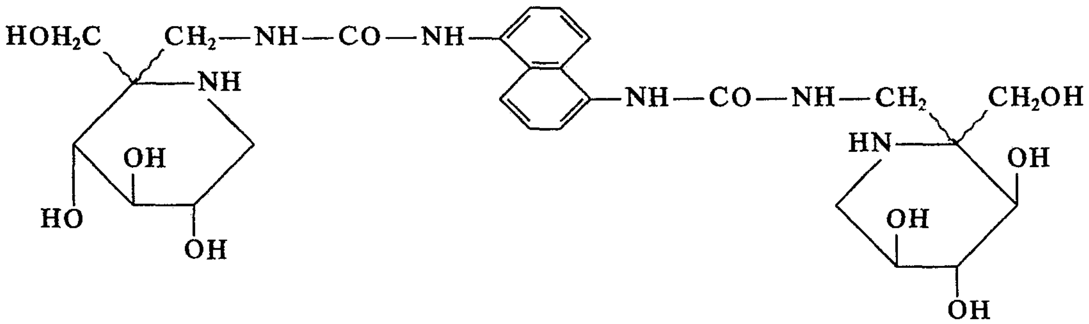

Schmelzpunkt: 232°C. $R_f$-Wert: 0,31

Beispiel 40

$N^1$,$N^4$-Bis-[5-(1,5-didesoxy-1,5-imino-D-glucityl)-methyl]-4-phenoxy-1,3-phenylen-bisharnstoff

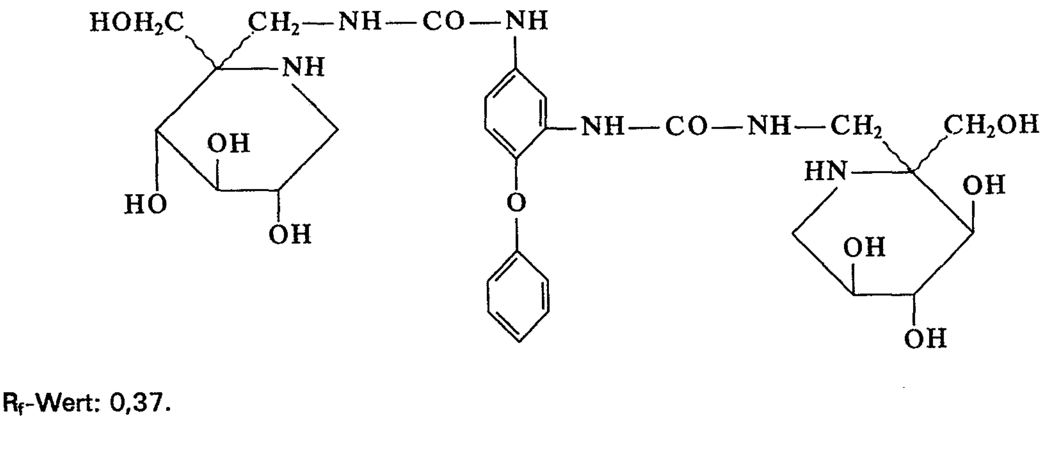

$R_f$-Wert: 0,37.

Beispiel 41

$N^1,N^4$-Bis-[5-(1,5-didesoxy-1,5-imino-D-glucityl)-methyl]-1,4-dibisureido-cyclohexan

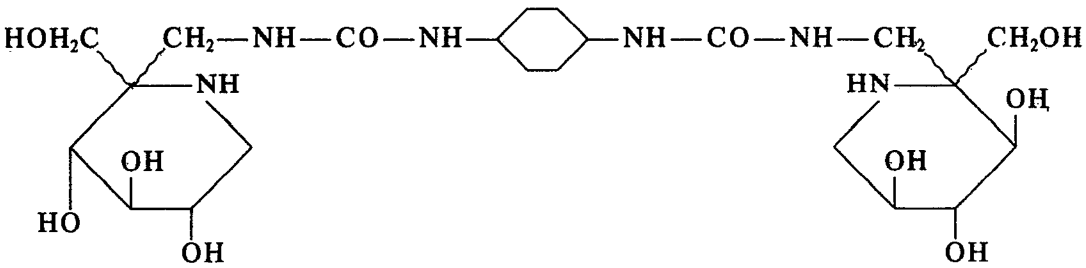

$R_f$-Wert: 0,30.

Diese Verbindung wurde analog zu Beispiel 10 hergestellt mit 1,6-Dibromo-hexadien-2,4 als Ausgangsmaterial:

Beispiel 42

1,6-[N,N'-Bis-(1',5'-didesoxy-1',5'-imino-D-glucit)-yl]-hexadien-2,4

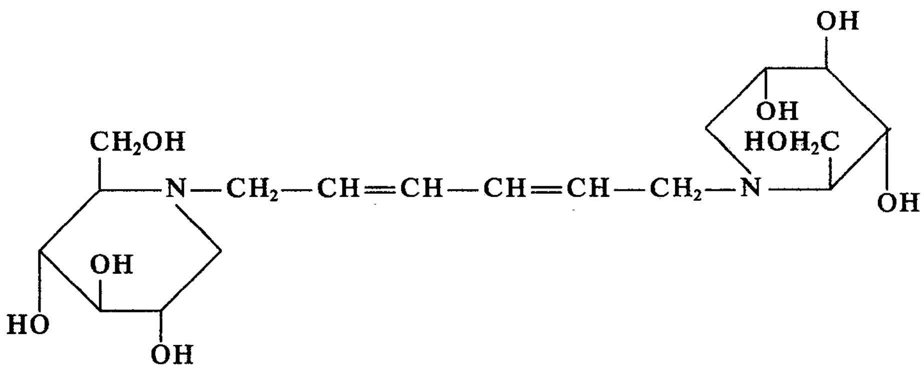

$R_f$-Wert: 0,48. $R_f$-Wert für 1-Desoxynojirimycin: 0,53.

Beispiel 43

1,4-Bis-{2-[N-(1',5'-didesoxy-1',5'-imino-D-glucit)-yl]-ethoxy}-benzol

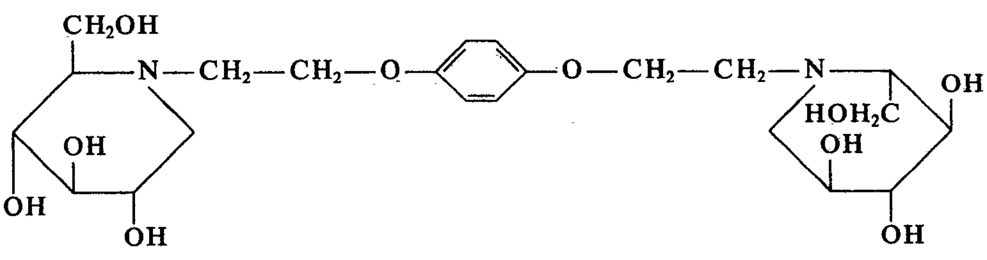

2,95 g 1-Desoxynojirimycin werden mit 5 g Kaliumcarbonat und 2,9 g Bis-(2-Bromethoxy)-benzol in 25 ml absolutem DMF 5 Stunden bei 100°C gerührt. Es wird heiß abgesaugt und das Filtrat eingeengt. Der Eindampfrückstand wird in wenig Methanol/Wasser gelöst und über eine Zellulose/Aceton-Säule gereinigt. Man erhält nach Umkristallisation aus Wasser 1,1 g farblose Kristalle vom Schmelzpunkt 226–28°C.

Analog wurden hergestellt:

Beispiel 44

4,4'-Bis-{2-[N-(1',5'-didesoxy-1',5'-imino-D-glucit)-yl]-ethoxy}-diphenyl

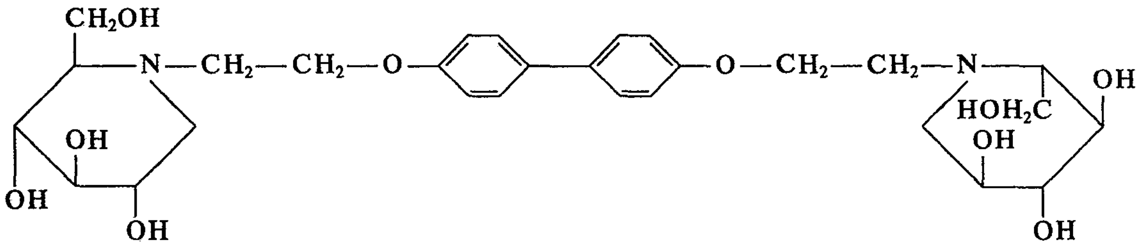

Schmelzpunkt: 238°C unter Zersetzung.

Beispiel 45

4,4'-Bis-{2-[N-(1',5'-didesoxy-1',5'-imino-D-glucit)-yl]-ethoxy}-benzoesäurebenzylamid

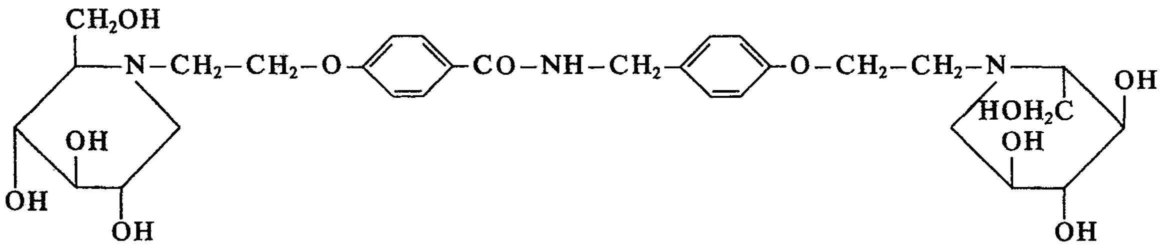

$R_f$-Wert: 0,68; $R_f$-Wert für 1-Desoxynojirimycin = 0,54 (Fließmittel: Methanol/Chloroform/25% Ammoniak 3 : 2 : 2).

Beispiel 46

N-{4-[2-(N'-<1',5'-didesoxy-1',5'-imino-D-glucit>-yl)-ethoxy]-benzyl}-1-desoxynojirimycin

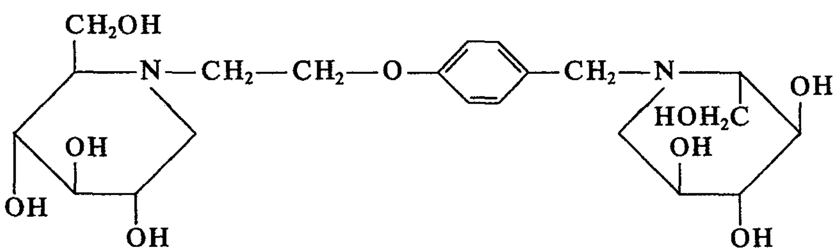

Diese Verbindung wurde hergestellt durch Umsetzung von 4-(β-Bromethoxy)-benzaldehyd mit 1-Desoxynojirimycin analog Beispiel 35 zu N-{4-[β-Bromethoxy]-benzyl}-1-desoxynojirimycin und anschließende Reaktion mit 1-Desoxynojirimycin unter den Bedingungen von Beispiel 43. Schmelzpunkt ab 155°C, Zersetzung.

Beispiel 47

1-{4-[2-(N<1',5'-didesoxy-1',5'-imino-D-glucit>-yl)-ethoxy]-benzamido-methyl}-1-desoxynojirimycin

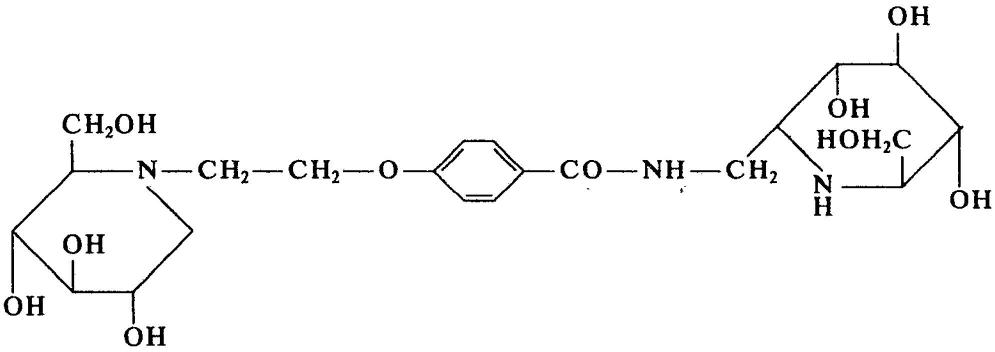

$R_f$-Wert: 0,45; $R_f$-Wert für 1-Desoxynojirimycin = 0,54. Fließmittel wie bei Beispiel 45.

Beispiel 48

5-{4-[2-(N<1',5'-didesoxy-1',5'-imino-D-glucit>-yl)-ethoxy]-benzamido-methyl}-1-desoxynojirimycin

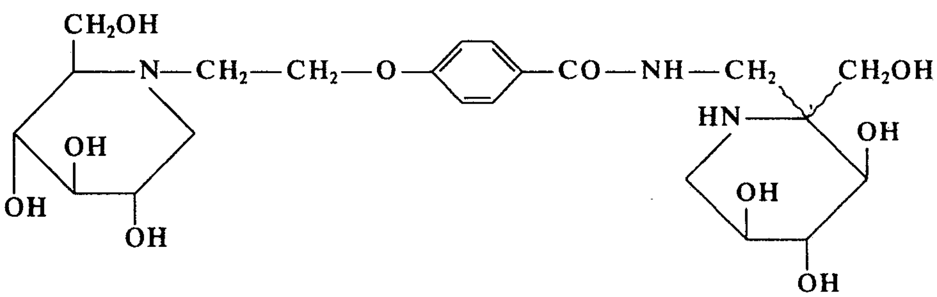

$R_f$-Wert: 0,47; $R_f$-Wert für 1-Desoxynojirimycin = 0,54. Fließmittel wie bei Beispiel 45.

**Patentansprüche**

1. Verbindungen der allgemeinen Formel

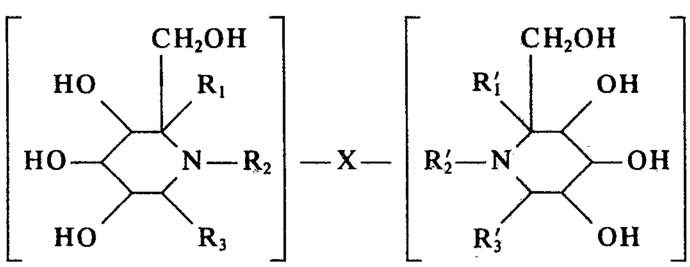

worin

$R_1$, $R'_1$, $R_3$ und $R'_3$ Wasserstoff oder eine direkte Bindung zu X,

$R_2$ und $R'_2$ Wasserstoff, $C_1-C_4$ Alkyl oder eine direkte Bindung zu X bedeuten, mit der Maßgabe, daß einer und nur einer der Reste $R_1$, $R_2$ und $R_3$ und einer und nur einer der Reste $R'_1$, $R'_2$ und $R'_3$ eine direkte Bindung zu X darstellen und

worin X der Formel

$$-(A)_m-(R_4)_n-(Y)_p-(R_5)_q-(B)_r-$$

entspricht, worin

m, n, p, q und r 0 oder 1 bedeuten, mit der Maßgabe, daß die Summe aus m und r 1 oder 2 ist und daß p 0 ist, wenn n und/oder q 0 sind und worin

A und B für

$CH_2$ $CH_2-NH$ $CH_2-NH-CO$ $CH_2-NHCONH$ $CH_2NHSO_2NH$
$CH_2-NH-SO_2$ $CH_2-NHCS-NH$ $CH_2-NH-COO$ $CH_2-NHCS$
$CH_2-NH-\underset{\underset{\displaystyle NH}{\|}}{C}-NH$ $CH_2-O$ $CO$ oder $COO$

stehen,
$R_4$ und $R_5$ unabhängig voneinander eine Gruppierung

$$-(R_{13})_s-(U)_t-(R_{14})_v$$

darstellen, in der $R_{13}$ und $R_{14}$ unabhängig voneinander $C_1-C_{18}$ Alkylen; $C_2-C_{18}$ Alkenylen mit bis zu drei Doppelbindungen; $C_2-C_{10}$ Alkinylen; $C_3-C_{10}$ Cycloalkylen; Naphthylen; Phenylen; Phenylen mit einem oder zwei Substituenten aus der Gruppe Phenoxy oder Methyl, einen heterocyclischen Rest mit 3–8 Ringgliedern und 1–4 Heteroatomen N, O und/oder S oder Kombinationen dieser Bedeutung darstellen,

U die Bedeutung von O, S, SO, $SO_2$, NH, CO, COO, CS, OCOO, NHCOO, CONH, NHCONH, NHCSNH, CH=N, $SO_2NH$ oder $NHSO_2NH$ hat,
s, t, v unabhängig voneinander für 0 oder 1 stehen,
Y die Bedeutung von U oder die Bedeutung von $R_{13}$ und $R_{14}$ hat.

2. Verbindungen nach Anspruch 1, worin

A $CH_2$ bedeutet, wenn die Verknüpfung von X über $R_2$ erfolgt, oder $CH_2-NHCO$, $CH_2NHSO_2$, $CH_2NHCOO$, $CH_2-NHCOO$ oder $CH_2-NHCONH$ bedeutet, wenn die Verknüpfung von X über $R_1$ oder $R_3$ erfolgt,
B $CH_2$ bedeutet, wenn die Verknüpfung von X über $R'_2$ erfolgt, oder $CH_2NHCO$, $CH_2NHSO_2$, $CH_2NHCOO$, $CH_2-NHCOO$ oder $CH_2NHCONH$ bedeutet, wenn die Verknüpfung von X über $R'_1$ oder $R'_2$ erfolgt,
$R_{13}$ und $R_{14}$ $C_1-C_{18}$ Alkyliden, $C_2-C_{18}$ Alkenyliden oder Phenylen,
U, Y, O, $SO_2$, CO, $CH_2$, S, SO, NH, CONH, NHCONH, NHCSNH, $SO_2NH$ oder CH=CH,
m und n 1 und r, p und p 0 oder 1 bedeuten.

3. Verfahren zur Herstellung von Verbindungen der allgemeinen Formel

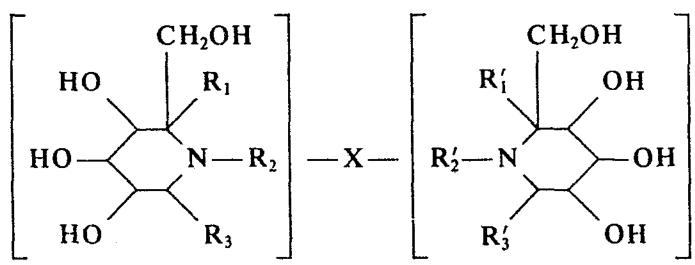

worin
$R_1$, $R'_1$, $R_3$ und $R'_3$ Wasserstoff oder eine direkte Bindung zu X,
$R_2$ und $R'_2$ Wasserstoff, $C_1-C_4$ Alkyl oder eine direkte Bindung zu X bedeuten, mit der Maßgabe, daß einer und nur einer der Reste $R_1$, $R_2$ und $R_3$ und einer und nur einer der Reste $R'_1$, $R'_2$ und $R'_3$ eine direkte Bindung zu X darstellen und
worin X der Formel

$$-(A)_m-(R_4)_n-(Y)_p-(R_5)_q-(B)_r-$$

entspricht, worin
m, n, p, q und r 0 oder 1 bedeuten, mit der Maßgabe, daß die Summe aus m und r 1 oder 2 ist und daß p 0 ist, wenn n und/oder q 0 sind und worin

A und B für

$CH_2$ $CH_2—NH$ $CH_2—NH—CO$ $CH_2—NHCONH$ $CH_2NHSO_2NH$
$CH_2—NH—SO_2$ $CH_2—NHCS—NH$ $CH_2—NH—COO$ $CH_2—NHCS$
$CH_2—NH—\underset{\underset{NH}{\|}}{C}—NH$ $CH_2—O$ CO oder COO

stehen,
$R_4$ und $R_5$ unabhängig voneinander eine Gruppierung

$$—(R_{13})_s—(U)_t—(R_{14})_v$$

darstellen, in der $R_{13}$ und $R_{14}$ unabhängig voneinander $C_1-C_{18}$ Alkylen; $C_2-C_{18}$ Alkenylen mit bis zu drei Doppelbindungen; $C_2-C_{10}$ Alkinylen; $C_3-C_{10}$ Cycloalkylen; Naphthylen; Phenylen; Phenylen mit einem oder zwei Substituenten aus der Gruppe Phenoxy oder Methyl, einen heterocyclischen Rest mit 3–8 Ringgliedern und 1–4 Heteroatomen N, O und/oder S oder Kombinationen dieser Bedeutung darstellen,

U die Bedeutung von O, S, SO, $SO_2$, NH, CO, COO, CS, OCOO, NHCOO, CONH, NHCONH, NHCSNH, CH=N, $SO_2NH$ oder $NHSO_2NH$ hat,
s, t, v unabhängig voneinander für 0 oder 1 stehen,
Y die Bedeutung von U oder die Bedeutung von $R_{13}$ und $R_{14}$ hat,

dadurch gekennzeichnet, daß man Verbindungen der Formel

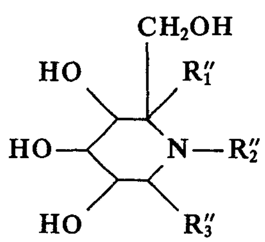

(III)

worin
$R_1''$ und $R_3''$ Wasserstoff oder $CH_2-NH_2$ und
$R_2''$ Wasserstoff oder $C_1-C_4$ Alkyl bedeuten, wobei die Zahl der $CH_2-NH_2$ 0 oder 1 ist und $R_2''$ Wasserstoff bedeutet, falls $R_1''$ und $R_3''$ Wasserstoff sind,
mit Dialdehyden der Formel IV

$$OCH—Z—CHO \qquad (IV)$$

worin
Z die zum Brückenglied X fehlenden Glieder bedeutet,
im Verhältnis 2 : 1 in Gegenwart eines Wasserstoffdonors umsetzt.

4. Verfahren zur Herstellung der Verbindungen gemäß Anspruch 3, dadurch gekennzeichnet, daß man Verbindungen der Formel III, in der $R_1''$, $R_2''$ und $R_3''$ die in Anspruch 3 angegebene Bedeutung haben, mit Alkylierungs- und Acylierungsmitteln der Formel

$$R_7—Z—R_7$$

worin
Z die in Anspruch 3 genannte Bedeutung hat und
$R_7$ eine in Alkylierungs- und Acylierungsmitteln übliche funktionelle Gruppe ist,
umsetzt.

5. Verfahren zur Herstellung der Verbindungen gemäß Anspruch 3, worin
$R_1$ und $R_1'$ Wasserstoff,
$R_2$ und $R_2'$ Wasserstoff oder $C_1-C_4$ Alkyl,
$R_3$ und $R_3'$ eine direkte Bindung zu X und
X ein $C_1-C_{18}$ Alkylenrest oder Phenylenrest ist,
dadurch gekennzeichnet, daß man Verbindungen der Formel

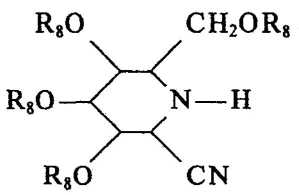

worin
$R_8$ eine übliche Schutzgruppe ist, mit Verbindungen der Formel

$$BrMg—Z'—MgBr$$

worin
Z' eine $C_1-C_{18}$ Alkylengruppe oder Phenylengruppe ist, umsetzt und anschließend die Schutzgruppen entfernt.

6. Verfahren zur Herstellung der Verbindungen gemäß Anspruch 3, dadurch gekennzeichnet, daß man Verbindungen der Formel III, in der $R_1''$, $R_2''$ und $R_3''$ die in Anspruch 3 angegebene Bedeutung haben, im Verhältnis 1 : 1 mit Verbindungen der Formel VIII

$$R_9—Z—R_{10} \qquad (VIII)$$

wobei
$R_9$ —CHO oder $R_7$ mit der in Anspruch 4 genannten Bedeutung und Z die in Anspruch 3 genannte Bedeutung haben und
$R_{10}$ $R_7$ oder eine in einen Rest $R_7$ überführbare Gruppe oder eine Aldehyd- oder Ketogruppe ist, gegebenenfalls in Gegenwart eines Wasserstoffdonors umsetzt und den Rest $R_{10}$ im Zwischenprodukt unter veränderten Reaktionsbedingungen oder nach Umwandlung in einen Rest $R_7$ mit einer weiteren Verbindung der Formel III gegebenenfalls in Gegenwart eines Wasserstoffdonors zur Reaktion bringt.

7. Arzneimittel, gekennzeichnet durch einen Gehalt an einer Verbindung gemäß Anspruch 1 und gegebenenfalls pharmazeutisch geeigneten Zusatzstoffen.

8. Verbindungen der Formel I gemäß Anspruch 1 zur Beeinflussung des Kohlenhydrat- und/oder Lipidstoffwechsels.

9. Verbindungen der Formel I gemäß Anspruch 1 zur besseren Futterverwertung und zur Beeinflussung des Fleisch-Fettverhältnisses zugunsten des Fleischanteils in der Tierernährung.

**Claims**

1. Compounds of the general formula

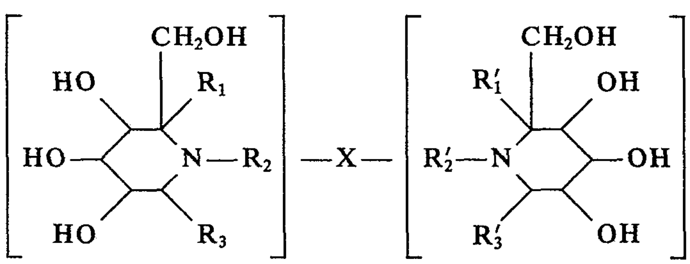

wherein
$R_1$, $R_1'$, $R_3$ and $R_3'$ denote hydrogen or a direct bond to X,
$R_2$ und $R_2'$ denote hydrogen, $C_1-C_4$ alkyl or a direct bond to X, with the proviso that one and only of the radicals $R_1$, $R_2$ and $R_3$ and one and only one of the radicals $R_1'$, $R_2'$ and $R_3'$ represent a direct bond to X, and
wherein X corresponds to the formula

$$—(A)_m—(R_4)_n—(Y)_p—(R_5)_q—(B)_r—$$

wherein
m, n, p, q and r denote 0 or 1, with the proviso that the sum of m and r is 1 or 2 and that p is 0 when n and/or q are 0 and
wherein
A and B represent

$CH_2$ $CH_2—NH$ $CH_2—NH—CO$ $CH_2—NHCONH$ $CH_2NHSO_2NH$
$CH_2—NH—SO_2$ $CH_2—NHCS—NH$ $CH_2—NH—COO$ $CH_2—NHCS$
$CH_2—NH—C(=NH)—NH$ $CH_2—O$ CO or COO

$R_4$ and $R_5$ independently of one another represent a grouping

$$—(R_{13})_s—(U)_t—(R_{14})_v$$

in which $R_{13}$ and $R_{14}$ independently of one another represent $C_1-C_{18}$ alkylene; $C_2-C_{18}$ alkenylene with up to three double bonds; $C_2-C_{10}$ alkinylene; $C_3-C_{10}$ cycloalkylene; napthylene; phenylene; phenylene with one or two substituents from the group comprising phenoxy and methyl, a heterocyclic radical with 3–8 ring members and 1–4 hetero atoms N, O and/or S or combinations of this meaning,

U signifies O, S, SO, $SO_2$, NH, CO, COO, CS, OCOO, NHCOO, CONH, NHCONH, NHCSNH, CH=N, $SO_2NH$ or $NHSO_2NH$,
s, t, and v independently of one another represent 0 or 1, and
Y has the meaning of U or the meaning of $R_{13}$ and $R_{14}$.

2. Compounds according to Claim 1, wherein

A denotes $CH_2$, if is linked via $R_2$, or $CH_2-NHCO$, $CH_2NHSO_2$, $CH_2NHCOO$, $CH_2-NHCOO$ or $CH_2-NHCONH$, if X is linked via $R_1$ or $R_3$,
B denotes $CH_2$, if X is linked via $R'_2$, or $CH_2NHCO$, $CH_2NHSO_2$, $CH_2NHCOO$, $CH_2-NHCOO$ or $CH_2NHCONH$ if X is linked via $R'_1$ or $R'_3$,
$R_{13}$ and $R_{14}$ denote $C_1-C_{18}$ alkylidene, $C_2-C_{18}$ alkenylidene or phenylene,
U and Y denote O, $SO_2$, CO, $CH_2$, S, SO, NH, CONH, NHCONH, NHCSNH, $SO_2NH$ or CH=CH and
m and n denote 1 and r, p and p denote 0 or 1.

3. Process for the preparation of compounds of the general formula

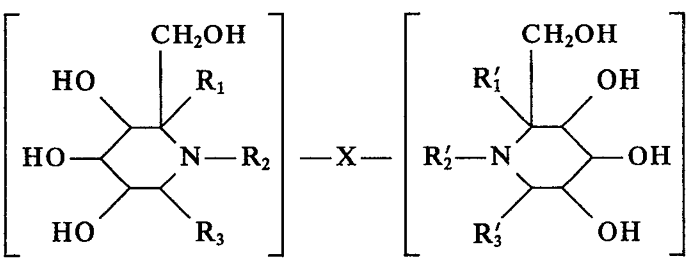

wherein
$R_1$, $R'_1$, $R_3$ and $R'_3$ denote hydrogen or a direct bond to X,
$R_2$ und $R'_2$ denote hydrogen, $C_1-C_4$ alkyl or a direct bond to X, with the proviso that one and only of the radicals $R_1$, $R_2$ and $R_3$ and one and only one of the radicals $R'_1$, $R'_2$ and $R'_3$ represent a direct bond to X and
wherein X corresponds to the formula

$$—(A)_m—(R_4)_n—(Y)_p—(R_5)_q—(B)_r—$$

wherein
m, n, p, q and r denote 0 or 1, with the proviso that the sum of m and r is 1 or 2 and that p is 0 if n and/or q are 0 and
wherein
A and B represent

$CH_2$ $CH_2—NH$ $CH_2—NH—CO$ $CH_2—NHCONH$ $CH_2NHSO_2NH$
$CH_2—NH—SO_2$ $CH_2—NHCS—NH$ $CH_2—NH—COO$ $CH_2—NHCS$
$CH_2—NH—C(=NH)—NH$ $CH_2—O$ CO or COO

$R_4$ and $R_5$ independently of one another represent a grouping

$$—(R_{13})_s—(U)_t—(R_{14})_v$$

in which $R_{13}$ and $R_{14}$ independently of one another represent $C_1-C_{18}$ alkylene; $C_2-C_{18}$ alkenylene with up to three double bonds; $C_2-C_{10}$ alkinylene; $C_3-C_{10}$ cycloalkylene; napthylene; phenylene; phenylene with one or two substituents from the group comprising phenoxy and methyl, a heterocyclic radical with 3–8 ring members and 1–4 hetero atoms N, O and/or S or combinations of this meaning,

U signifies O, S, SO, $SO_2$, NH, CO, COO, CS, OCOO, NHCOO, CONH, NHCONH, NHCSNH, CH=N, $SO_2NH$ or $NHSO_2NH$,

s, t, and v independently of one another represent 0 or 1, and

Y has the meaning of U or the meaning of $R_{13}$ and $R_{14}$,

characterised in that compounds of the formula

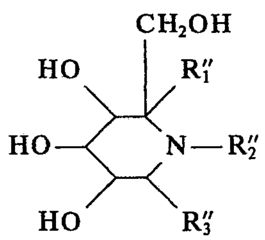

(III)

wherein

$R_1''$ and $R_3''$ denote hydrogen or $CH_2-NH_2$ and

$R_2''$ denotes hydrogen or $C_1-C_4$ alkyl, the number of $CH_2-NH_2$ being 0 or 1 and $R_2''$ denoting hydrogen if $R_1''$ and $R_3''$ are hydrogen,

are reacted with dialdehyds of the formula IV

$$OCH—Z—CHO \qquad (IV)$$

wherein

Z denotes the missing members of the bridge member X, in the ratio 2 : 1 in the presence of a hydrogen donor.

4. Process für the preparation of compounds according to Claim 3, characterised in that compounds of the formula III in which $R_1''$, $R_2''$ and $R_3''$ have the meaning indicated in Claim 3, are reacted with alkylating agents or acylating agents of the formula

$$R_7—Z—R_7$$

wherein

Z has the meaning indicated in Claim 3 and

$R_7$ is a functional group customary in alkylating agents or acylating agents.

5. Process for the preparation of the compounds according to Claim 3, wherein

$R_1$ and $R_1'$ is hydrogen,

$R_2$ und $R_2'$ ist hydrogen or $C_1-C_4$ alkyl,

$R_3$ and $R_3'$ is a direct bond to X and

X is a $C_1-C_{18}$ alkylene radical or phenylene radical,

characterised in that compounds of the formula

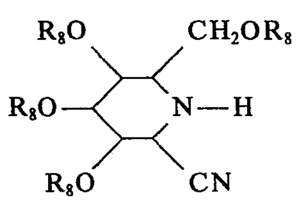

wherein

$R_8$ is a customary protective group,

are reacted with compounds of the formula

$$BrMg—Z'—MgBr$$

wherein

Z′ is a $C_1-C_{18}$ alkylene group or phenylene group and the protective groups are then removed.

6. Process for the preparation of the compounds according to Claim 3, characterised in that compounds of the formula III in which $R_1''$, $R_2''$ and $R_3''$ have the meaning indicated in Claim 3, are reacted in a ratio 1 : 1 with compounds of the formula VIII

$$R_9—Z—R_{10} \qquad (VIII)$$

wherein

$R_9$ signifies $-CHO$ or $R_7$ with the meaning indicated in Claim 4 and

Z has the meaning indicated in Claim 3 and

$R_{10}$ is $R_7$ or a group which can be converted into a radical $R_7$ or an aldehyde or keto group, if appropriate in the presence of a hydrogen donor and the radical $R_{10}$ in the intermediate product is reacted, under changed reaction conditions or after conversion into a radical $R_7$, with a further compound of the formula III, if appropriate in the presence of a hydrogen donor.

7. Medicaments, characterised in that they contain a compound according to Claim 1 and, if appropriate, pharmaceutically suitable additives.

8. Compounds of the formula I according to Claim 1 for influencing the carbohydrate and/or lipid metabolism.

9. Compounds of the formula I according to Claim 1 for the better utilisation of feed and for influencing the lean meat/fat ratio in favour of the proportion of lean meat in animal nutrition.

**Revendications**

1. Composés de formule générale:

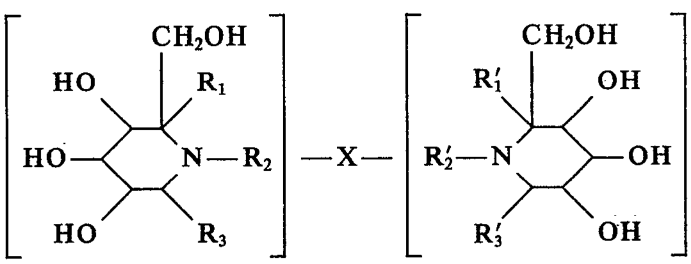

dans laquelle

$R_1$, $R_1'$, $R_3$ et $R_3'$ représentent chacun un atome d'hydrogène ou une liaison directe avec X,

$R_2$ et $R_2'$ représentent chacun un atome d'hydrogène, un group alkyle en $C_1-C_4$ ou une liaison directe avec X, à condition qu'un et un seul des radicaux $R_1$, $R_2$ et $R_3$, ainsi qu'un et un seul des radicaux $R_1'$, $R_2'$ et $R_3'$ représentent une liaison directe avec X, et

X répond à la formule:

$$—(A)_m—(R_4)_n—(Y)_p—(R_5)_q—(B)_r—$$

où m, n, p, q et r représentent chacun 0 ou 1, à condition que la somme de m et r soit 1 ou 2 et que p représente 0 lorsque n et/ou q représentent 0, tandis que

A et B représentent chacun

$CH_2$ $CH_2—NH$ $CH_2—NH—CO$ $CH_2—NHCONH$ $CH_2NHSO_2NH$

$CH_2—NH—SO_2$ $CH_2—NHCS—NH$ $CH_2—NH—COO$ $CH_2—NHCS$

$CH_2—NH—C(=NH)—NHCH_2—O$ CO oder COO

$R_4$ et $R_5$ représentent chacun indépendamment l'un de l'autre un groupement

$$—(R_{13})_s—(U)_t—(R_{14})_v$$

où $R_{13}$ et $R_{14}$ représentent chacun indépendamment l'un de l'autre un groupe alkylène en $C_1-C_{18}$; un groupe alcénylène en $C_2-C_{18}$ contenant jusqu'à trois doubles liaisons; un groupe alcinylène en $C_2-C_{10}$; un groupe cycloalkylène en $C_3-C_{10}$; un groupe naphtylène, un groupe phénylène; un groupe phénylène contenant un ou deux substituants choisis parmi le groupe comprenant un groupe phénoxy

ou un groupe méthyle; un radical hétérocyclique contenant 3 à 8 termes cycliques et 1—4 hétéroatomes N, O et/ou S ou des combinaisons de cette signification,

U représente O, S, SO, $SO_2$, NH, CO, COO, CS, OCOO, NHCOO, CONH, NHCONH, NHCSNH, CH=N, $SO_2NH$ ou $NHSO_2NH$,
s, t et v représentent chacun indépendamment l'un de l'autre 0 ou 1,
Y a la signification de U ou la signification de $R_{13}$ et $R_{14}$.

2. Composés suivant la revendication 1, dans lesquels:

A représente $CH_2$ lorsque la liaison de X a lieu à l'intervention de $R_2$ ou $CH_2$—NHCO, $CH_2NHSO_2$, $CH_2NHCOO$, $CH_2$—NHCOO ou $CH_2$—NHCONH lorsque la liaison de X a lieu à l'intervention de $R_1$ ou de $R_3$,
B représente $CH_2$ lorsque la liaison de X a lieu à l'intervention de $R'_2$, ou $CH_2NHCO$, $CH_2NHSO_2$, $CH_2NHCOO$, $CH_2$—NHCOO ou $CH_2NHCONH$ lorsque la liaison de X a lieu à l'intervention de $R'_1$ ou de $R'_3$,
$R_{13}$ et $R_{14}$ étant chacun un groupe alkylidène en $C_1—C_{18}$, un groupe alcénylidène en $C_2—C_{18}$ ou un groupe phénylène,
U représente Y, O, $SO_2$, CO, $CH_2$, S, SO, NH, CONH, NHCONH, NHCSNH, $SO_2NH$ ou CH=CH,
m et n représentent 1, tandis que r, p et q représentent 0 ou 1.

3. Procédé de préparation de composés de formule générale:

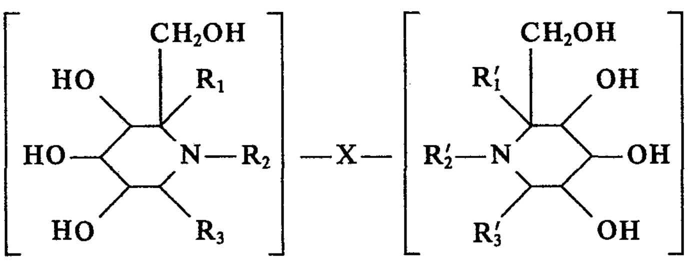

dans laquelle
$R_1$, $R'_1$, $R_3$ et $R'_3$ représentent chacun un atome d'hydrogène ou une liaison directe avec X,
$R_2$ et $R'_2$ représentent chacun un atome d'hydrogène, un groupe alkyle en $C_1—C_4$ou une liaison directe avec X, à condition qu'un et un seul des radicaux $R_1$, $R_2$ et $R_3$, ainsi qu'un et un seul des radicaux $R'_1$, $R'_2$ et $R'_3$ représentent une liaison directe avec X, et
X répond à la formule:

$$—(A)_m—(R_4)_n—(Y)_p—(R_5)_q—(B)_r—$$

dans laquelle
m, n, p, q et r représentent chacun 0 ou 1, à condition que la somme de m et r soit 1 ou 2 et que p représente 0 lorsque n et/ou q représentent 0, tandis que
A et B représentent chacun

$CH_2$ $CH_2—NH$ $CH_2—NH—CO$ $CH_2—NHCONH$ $CH_2NHSO_2NH$
$CH_2—NH—SO_2$ $CH_2—NHCS—NH$ $CH_2—NH—COO$ $CH_2—NHCS$
$CH_2—NH—\underset{\underset{NH}{\|}}{C}—NHCH_2—O$ CO ou COO

$R_4$ et $R_5$ représentent chacun indépendamment l'un de l'autre un groupement

$$—(R_{13})_s—(U)_t—(R_{14})_v$$

où $R_{13}$ et $R_{14}$ représentent chacun indépendamment l'un de l'autre un groupe alkylène en $C_1—C_{18}$; un groupe alcénylène en $C_2—C_{18}$ contenant jusqu'à 3 doubles liaisons; un groupe alcinylène en $C_2—C_{10}$, un groupe cycloalkylène en $C_3—C_{10}$; un groupe naphtylène; un groupe phénylène, un groupe phénylène contenant un ou deux substituants choisis parmi le groupe comprenant un groupe phénoxy ou un groupe méthyle; un radical hétérocyclique contenant 3 à 8 termes cycliques et 1 à 4 hétéroatomes N, O et/ou S ou des combinaisons de cette signification,

U représente O, S, SO, $SO_2$, NH, CO, COO, CS, OCOO, NHCOO, CONH, NHCONH, NHCSNH, CH=N, $SO_2NH$ ou $NHSO_2NH$,
s, t et v représentent chacun indépendamment l'un de l'autre 0 ou 1,
Y a la signification de U ou la signification de $R_{13}$ et $R_{14}$,

caractérisé en ce qu'on fait réagir des composés de formule:

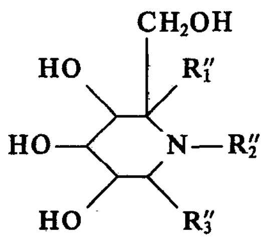

(III)

dans laquelle
$R_1''$ et $R_3''$ représentent chacun un atome d'hydrogène ou $CH_2-NH_2$ et
$R_2''$ représente un atome d'hydrogène ou un groupe alkyle en $C_1-C_4$, le nombre de $CH_2-NH_2$ étant 0 ou 1 et $R_2''$ représentant un atome d'hydrogène si $R_1''$ et $R_3''$ représentent chacun un atome d'hydrogène,
avec des dialdéhydes de formule IV:

$$OCH—Z—CHO \quad (IV)$$

dans laquelle
Z représente les termes manquants pour le terme en pont X,
dans le rapport de 2:1, en présence d'un donneur d'hydrogène.

4. Procédé de préparation des composés suivant la revendication 3, caractérisé en ce qu'on fait réagir des composés de formule III dans laquelle $R_1''$, $R_2''$ et $R_3''$ ont la signification indiquée dans la revendication 3, avec des agents d'alkylation et d'acylation de formule:

$$R_7—Z—R_7$$

dans laquelle
Z a la signification indiquée dans la revendication 3 et
$R_7$ représente un groupe fonctionnel habituel dans les agents d'alkylation et d'acylation.

5. Procédé de préparation des composés suivant la revendication 3, dans lesquels
$R_1$ et $R_1'$ représentent chacun un atome d'hydrogène,
$R_2$ et $R_2'$ représentent chacun un atome d'hydrogène ou un groupe alkyle en $C_1-C_4$,
$R_3$ et $R_3'$ représentent chacun une liaison directe avec X, et
X représente un groupe alkylène en $C_1-C_{18}$ ou un groupe phénylène,
caractérisé en ce qu'on fait réagir des composés de formule:

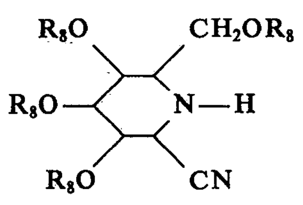

dans laquelle
$R_8$ représente un groupe protecteur habituel,
avec des composés de formule:

$$BrMg—Z'—MgBr$$

dans laquelle
Z' représente un groupe alkylène en $C_1-C_{18}$ ou un groupe phénylène,
puis on élimine les groupes protecteurs.

6. Procédé de préparation des composés suivant la revendication 3, caractérisé en ce qu'on fait réagir des composés de formule III dans laquelle $R_1''$, $R_2''$ et $R_3''$ ont la signification indiquée dans la revendication 3,

dans le rapport de 1 : 1, avec des composés de formule VIII:

$$R_9—Z—R_{10} \qquad (VIII)$$

$R_9$ représentant —CHO ou $R_7$ avec la signification indiquée dans la revendication 4 et Z ayant la signification indiquée dans la revendication 3, tandis que

$R_{10}$ représente $R_7$, un groupe transformable en un radical $R_7$, un groupe aldéhyde ou un groupe céto, éventuellement en présence d'un donneur d'hydrogène et l'on fait réagir le radical $R_{10}$ du produit intermédiaire dans des conditions réactionnelles modifiées ou après transformation en un radical $R_7$ avec un autre composé de formule III, éventuellement en présence d'un donneur d'hydrogène.

7. Médicaments, caractérisés en ce qu'ils contiennent un composé suivant la revendication 1 et éventuellement des additifs pharmaceutiquement appropriés.

8. Composés de formule I suivant la revendication 1 en vue d'influencer le métabolisme des hydrates de carbone et/ou des lipides.

9. Composés de formule I suivant la revendication 1 en vue de mieux valoriser le fourrage et influencer le rapport viande/graisse au profit de la fraction de viande dans l'alimentation des animaux.